# EUROPEAN PATENT SPECIFICATION

(11) **EP 3 070 138 B1**
(45) Date of publication and mention of the grant of the patent: **02.01.2019**
(21) Application number: 14862260.8
(22) Date of filing: 11.11.2014
(51) Int. Cl.: A61Q 1/02, A61Q 1/10, A61K 8/81, A61K 8/02, D21H 17/67

(54) **ULTRAVIOLET SCATTERING AGENT AND APPLICATION THEREFOR**
ULTRAVIOLETTSTREUUNGSMITTEL UND ANWENDUNG DAFÜR
AGENT DE DIFFUSION DU RAYONNEMENT ULTRAVIOLET ET SON APPLICATION

(30) Priority: 14.11.2013 JP 2013235873
(43) Date of publication of application: 21.09.2016
(73) Proprietor: Nisshinbo Holdings Inc., Tokyo 103-8650 (JP)
(72) Inventor: HAYAKAWA Kazutoshi, Chiba-shi Chiba 267-0056 (JP); MATSUZAKA Erina, Chiba-shi Chiba 267-0056 (JP); YAMADA Goki, Chiba-shi Chiba 267-0056 (JP); HASHIBA Toshifumi, Chiba-shi Chiba 267-0056 (JP)
(74) Representative: Bailey, Sam Rogerson
(86) International application number: PCT/JP2014/079816
(87) International publication number: WO 2015/072443

(56) References cited:
- JP-A- H11 180 827
- JP-A- 2004 115 342
- JP-A- 2009 001 759
- JP-A- 2009 235 355
- US-A1- 2007 054 123
- DATABASE WPI Week 200971 Thomson Scientific, London, GB; AN 2009-P75368 XP002769948, & JP 2009 235355 A (NISSHINBO IND INC) 15 October 2009 (2009-10-15)

## Description

### TECHNICAL FIELD

This invention relates to an ultraviolet (UV) scattering agent and to applications thereof.

### BACKGROUND ART

Micron-size polymer particles and inorganic particles are used as fillers and specimens in a variety of fields, such as electrical and electronic materials, optical materials, paints, inks, construction materials, biological and pharmaceutical materials, and cosmetics. In recent years, active research has been carried out particularly on fine particles of unusual shapes differing from the spherical; with diverse properties, including optical characteristics and tactile feel, being imparted to such particles, new applications are constantly being developed. The inventors have been working on the development of elliptical or needle-like particles of high aspect ratio, and have already developed particles which are superior to conventional spherical particles in terms of such properties as hiding power, light scattering ability and tactile qualities (Patent Documents 1 to 5).

Additives such as UV absorbers and UV scattering (diffusing) agents are used in a variety of fields, such as cosmetics, paints and inks, construction materials, interior design and plastic products, to prevent adverse effects by UV on the human body and to prevent the UV deterioration of shaped articles, compositions and the like.

Increasing the amount of UV absorber in a formulation generally increases its effect, but because the UV-shielding mechanism is primarily absorption, one drawback is that only UV within a specific wavelength region can be shielded. In addition, problems with the durability of the effect sometimes arise. Also, when UV absorbers are used in cosmetics, for example, they may have undesirable effects on the body, such as causing rough, dry skin or rashes in people with sensitive skin.

On the other hand, UV scattering agents composed of inorganic particles such as zinc oxide or titanium oxide generally have a high UV scattering effect. However, maintaining a dispersion with the particles in a primary particle state is difficult; instead, such particles are often present as agglomerates, as a result of which the hiding power becomes too high. Another drawback is that the addition of even a small amount leads to a loss of glossiness.

Inorganic materials have a higher specific gravity than organic substances and, when used in films and shaped articles, are not readily compatible with the resin, which can make it difficult to reduce product weight and may give rise to performance problems such as a tendency for cracking. Moreover, in cosmetics, a large amount of surfactant must sometimes be used to maintain the dispersion, but using a large amount of surfactant is undesirable from the standpoint of preventing rough, dry skin and skin aging.

### PRIOR ART DOCUMENTS

### PATENT DOCUMENTS

Patent Document 1: JP-A 2005-247979
Patent Document 2: JP-A 2006-104401
Patent Document 3: JP-A 2007-070372
Patent Document 4: JP-A 2009-235353
Patent Document 5: JP-A 2009-235355

US 2007/054123 A1 discloses an organic polymer particle which has an oval-spherical shape and a single continuous curved surface which allows achievement of optical characteristics such as light scattering and light collecting properties, and friction characteristics such as slip. The particle has an aspect ratio or 1.8 or more and is composed of a polymer of a first and second organic monomer. The first organic monomer has an ionic functional group and a polymerizable group, and the second organic monomer is polymerizable with the first organic monomer.

JP 2009 235355 A discloses an elliptical or needle-like polymer particle capable of enhancing an optical characteristic such as light diffusability and light convergency, a frictional characteristic such as slidability. The particle has a defined aspect ratio of 1.8 or more and a fine irregularity on a surface.

### SUMMARY OF THE INVENTION

### PROBLEMS TO BE SOLVED BY THE INVENTION

It is therefore an object of the invention to provide a UV scattering agent which is lightweight, is endowed with outstanding light-scattering properties, feel and flowability, and moreover has excellent safety, and can therefore be used as a UV-cutting (shielding) additive in, for example, cosmetics, paints, inks, and films and sheets.

### MEANS FOR SOLVING THE PROBLEMS

The inventors have conducted extensive investigations, as a result of which they have discovered that elliptical or needle-like polymer particles of a given shape have a UV-scattering effect.

Accordingly, the invention provides the following UV scattering agent and applications thereof.
1. An ultraviolet scattering agent characterized by comprising at least one type of elliptical or needle-like polymer particle A, wherein
   (1) a projected two-dimensional image obtained by irradiating the particle with light from a direction perpendicular to a long axis of the particle has a length (L) the average (L_{AV}) of which is 0.1 to 80 µm,
   (2) a projected two-dimensional image obtained by irradiating the particle with light from a direction perpendicular to a long axis of the particle has a breadth (D) the average (D_{AV}) of which is 0.05 to 40 µm, and
   (3) the average (P_{AV}) of the aspect ratio (L/D) calculated from the length (L) and breadth (D) is from 2 to 30, characterized in that the ultraviolet scattering agent further comprises at least one type of particle B which has
   (4) a shape differing from that of polymer particle A and a volume mean particle size (MV_{B}) that satisfies the condition 1/5 × D_{AV} ≤ MV_{B} ≤ L_{AV}.
2. The UV scattering agent of 1 above, wherein polymer particle A is made of at least one type of resin selected from the group consisting of styrene resins, (meth)acrylic resins, vinyl ester resins, poly-N-vinyl compound resins, polyolefin resins, polyldiene resins, polyester resins, silicone resins, polyurethane resins, polyamide resins, polyimide resins, epoxy resins, polyvinyl butyral resins, phenolic resins, amino resins, oxazoline resins and carbodiimide resins.
3. The UV scattering agent of 1 or 2 above, wherein polymer particle A is made of a (co)polymer comprising at least one type of monomer selected from the group consisting of styrenes, (meth)acrylic acids, (meth)acrylic esters, vinyl esters, N-vinyl compounds, olefins, fluorinated olefins and conjugated dienes.
4. The UV scattering agent of 3 above, wherein particle B is a polymer particle.
5**.** The UV scattering agent of any one of 1 to 4 above, wherein particle B is spherical or substantially spherical.
6. The UV scattering agent of any one of 1 to 5 above, wherein the weight ratio of polymer particle A to particle B is between 99:1 and 10:90.
7. A resin composition comprising the UV scattering agent of any one of 1 to 6 above.
8. A dispersion comprising the UV scattering agent of any one of 1 to 6 above.
9. A paint comprising the UV scattering agent of any one of 1 to 6 above.
10. An ink comprising the scattering agent of any one of 1 to 6 above.
11. A cosmetic composition comprising the UV scattering agent of any one of 1 to 6 above.
12. A shaped article comprising the UV scattering agent of any one of 1 to 6 above.
13. The shaped article of 12 above which is a film, sheet or paper.
14. A method for imparting UV shielding properties by adding the UV scattering agent of any one of 1 to 6 above to a transparent or translucent resin, water or a volatile oil.

### ADVANTAGEOUS EFFECTS OF THE INVENTION

The UV scattering agent of the invention, by being composed of polymer particles, is lightweight and has excellent light scattering properties, tactile qualities and flowability. In addition, it also has an excellent safety and can be suitably used as a UV-cutting additive.

### BRIEF DESCRIPTION OF THE DIAGRAM

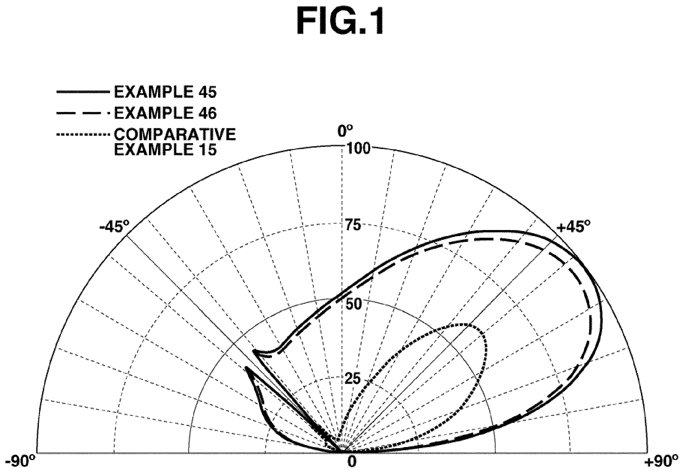

FIG. 1 is a diagram showing the light scattering distribution of reflected light measured in Examples 45 and 46 and Comparative Example 15.

### EMBODIMENT FOR CARRYING OUT THE INVENTION

### [UV Scattering Agent]

The UV scattering agent of the invention includes at least one type of elliptical or needle-like polymer particle A, wherein (1) a projected two-dimensional image obtained by irradiating the particle with light from a direction perpendicular to a long axis of the particle has a length (L) the average (L_{AV}) of which is 0.1 to 80 µm, (2) a projected two-dimensional image obtained by irradiating the particle with light from a direction perpendicular to a long axis of the particle has a breadth (D) the average (D_{AV}) of which is 0.05 to 40 µm, and (3) the average (P_{AV}) of the aspect ratio (L/D) calculated from the length (L) and breadth (D) is from 2 to 30.

The average length L_{AV} of polymer particle A is from 0.1 to 80 µm, preferably 0.2 to 50 µm, more preferably 1.0 to 30 µm, and even more preferably 2 to 20 µm. When L_{AV} is greater than 80 µm, the decrease in specific surface area per unit tends to be accompanied by a marked decrease in the UV scattering effect. On the other hand, when L_{AV} is less than 0.1 µm, the breadth of the particles is small, allowing UV light to pass through and reducing the UV scattering effect.

The average breadth D_{AV} of polymer particle A is from 0.05 to 40 µm, preferably 0.1 to 25 µm, more preferably 0.5 to 15 µm, and even more preferably 1 to 10 µm. When D_{AV} is greater than 40 µm, the decrease in specific surface area per unit tends to be accompanied by a marked decrease in the UV scattering effect. On the other hand, when D_{AV} is less than 0.05 µm, UV light passes through, reducing the UV scattering effect.

The average aspect ratio P_{AV} of polymer particle A is from 2 to 30 µm, preferably 3 to 25 µm, more preferably 3.5 to 20 µm, and most preferably 4 to 18 µm. When P_{AV} is greater than 30 µm, the particles tend to orient themselves, as a result of which optical characteristics such as a UV scattering effect, light scattering properties in the visible light region and light reflectivity cannot be stably obtained. On the other hand, when P_{AV} is less than 2, all that is obtained is a UV scattering effect of the same degree as that of spherical particles made of the same ingredients, and so the effects leave something to be desired.

Also, the volume mean particle size (MV_{A}) of the polymer particle A is preferably from 0.06 to 50 µm, more preferably 0.1 to 30 µm, and even more preferably 0.5 to 20 µm. When MV_{A} is greater than 50 µm, the decrease in specific surface area per unit tends to be accompanied by a decrease in the UV scattering effect. On the other hand, when MV_{A} is less than 0.06 µm, UV light may pass through, reducing the UV scattering effect.

In this invention, "volume mean particle size" is a measured value obtained by the laser scattering diffraction method and refers to, in the case of elliptical or needle-like particles or unusually shaped particles, the mean diameter of spheres having the same volumes as the particles (i.e., mean sphere-equivalent diameter of the particles).

The polymer particle A is preferably made of at least one type of resin selected from among styrene resins, (meth)acrylic resins, vinyl ester resins, poly-N-vinyl compound resins, polyolefin resins, polydiene resins, polyester resins, silicone resins, polyurethane resins, polyamide resins, polyimide resins, epoxy resins, polyvinyl butyral resins, phenolic resins, amino resins, oxazoline resins and carbodiimide resins. The resin in this case may be a homopolymer or a copolymer. For example, "styrene resins" are resins in which styrenes serve as the main structural units, and include not only styrene homopolymers, but also styrene copolymers and copolymers of styrenes and other monomers.

Examples of styrene resins include (co)polymers of styrenes, styrene-(meth)acrylic acid copolymers, styrene-(meth)acrylic ester copolymers, acrylonitrile-styrene copolymers, acrylonitrile-chlorinated polyethylene-styrene copolymers, styrene-maleic anhydride copolymers and modified forms of these; and copolymers of styrene with olefins or conjugated dienes, such as styrene-butadiene block copolymers (SBR), styrene-butadiene-styrene block copolymers (SBS), hydrogenated styrene-butadiene-styrene block copolymers (SEBS), styrene-isoprene block copolymers (SIR), styrene-isoprene-styrene block copolymers (SIS) and hydrogenated styrene-isoprene-styrene block copolymers (SEPS).

Examples of (meth)acrylic resins include (meth)acrylic acid (co)polymers, (meth)acrylic ester (co)polymers, (meth)acrylic acid-(meth)acrylic ester copolymers, vinyl ester-(meth)acrylic acid copolymers, vinyl ester-(meth)acrylic ester copolymers, olefin-(meth)acrylic acid copolymers such as ethylene-acrylic acid copolymers, olefin-(meth)acrylic ester copolymers such as ethylene-acrylic ester copolymers, N-vinyl compound-(meth)acrylic acid copolymers, N-vinyl compound-(meth)acrylic ester copolymers, conjugated diene-(meth)acrylic acid copolymers and conjugated diene-(meth)acrylic ester copolymers.

Examples of vinyl ester resins include (co)polymers of vinyl esters, olefin-vinyl ester copolymers such as ethylene-vinyl acetate copolymers, and vinyl ester-conjugated diene copolymers. Examples of poly-N-vinyl compound resins include (co)polymers of N-vinyl compounds, copolymers of olefin-N-vinyl compounds and copolymers of conjugated diene-N-vinyl compounds. Examples of polyolefin resins include polyolefin, polyfluorinated olefins, copolymers of olefins and/or fluorinated polyolefins, and olefin-conjugated diene copolymers. Examples of polydiene resins include (co)polymers of conjugated dienes.

Resins made up of unsaturated monomers, such as the above styrene resins, (meth)acrylic resins, vinyl ester resins, poly-N-vinyl compound resins, polyolefin resins and polydiene resins, may be prepared as copolymers of two or more of these types, as suitable for the intended application and purposes.

The polyester resins are not particularly limited, and are exemplified by polyester resins in which the primary acid component is, for example, terephthalic acid or dimethyl terephthalate and the primary glycol component is at least one alkylene glycol selected from among ethylene glycol, diethylene glycol, trimethylene glycol and butylene glycol; and by polylactic acids. Specific examples include polyethylene terephthalate, polyethylene naphthalate, polybutylene terephthalate, polybutylene naphthalate, polytrimethylene terephthalate, polycyclohexylene dimethylene terephthalate, polycyclohexylene dimethylene naphthalate, polybutylene terephthalate, polybutylene naphthalate and polylactic acid.

The silicone resins are not particularly limited, provided they include silicon-silicon bonds, silicon-carbon bonds, siloxane bonds or silicon-nitrogen bonds on the molecular chain. Specific examples include polysiloxane, polycarbosilane and polysilazane.

The polyurethane resins are exemplified by polyurethane resins obtained by polymerizing polyols and polyisocyanates. Examples of the polyol include ethylene glycol, diethylene glycol, propylene glycol, dipropylene glycol, glycerol, 1,1,1-trimethylolpropane, 1,2,5-hexanetriol, 1,3-butanediol, 1,4-butanediol, 4,4'-dihydroxyphenylpropane, 4,4'-dihydroxyphenylmethane and pentaerythritol. Examples of the polyisocyanate include 4-tolylene diisocyanate, 2,6-tolylene diisocyanate, 4,4'-diphenylmethane diisocyanate, 2,4'-diphenylmethane diisocyanate, p-phenylene diisocyanate, isophorone diisocyanate and xylylene diisocyanate.

Examples of polyamide resins include polyamide resins obtained by polycondensing a dicarboxylic acid such as adipic acid, heptanedicarboxylic acid, octanedicarboxylic acid, nonanedicarboxylic acid, undecanedicarboxylic acid or dodecanedicarboxylic acid with a diamine such as tetramethylenediamine, hexamethylenediamine, octamethylenediamine, nonamethylenediamine, undecamethylenediamine or dodecamethylenediamine. Other examples include polyamide resins obtained by the ring-opening polymerization of lactams such as α-pyrrolidone, ε-caprolactam, ω-laurolactam or ε-enantholactam. Specific examples include nylon 6, nylon 11, nylon 12, nylon 6,6 and nylon 6,T.

Examples of polyimide resins include polyimide resins obtained by polymerizing a diamine such as o-phenylenediamine, m-phenylenediamine, p-phenylenediamine, 4,4'-diaminodiphenyl ether, 1,4-bis(aminomethyl)cyclohexane, 1,3-bis(aminomethyl)cyclohexane, 1,3-propanediamine, 1,4-butanediamine, 1,5-pentanediamine or 1,6-hexanediamine with a tetracarboxylic dianhydride such as 4,4'-hexafluoropropylidene bis(phthalic dianhydride), 4,4'-biphthalic dianhydride, diphenyl-2,3,3',4'-tetracarboxylic dianhydride, diphenyl-2,2',3,3'-tetracarboxylic dianhydride or pyromellitic dianhydride.

Examples of epoxy resins include polyepoxides, aromatic polyepoxy compounds, glycidyl ethers of polyphenols, glycidyl esters of polyphenols, glycidyl aromatic polyamines, alicyclic polyepoxy compounds, aliphatic polyepoxy compounds and polyglycidyl esters of polyfunctional fatty acids. Of these, aromatic aliphatic polyepoxy compounds and aromatic polyepoxy compounds are preferred.

Examples of polyvinyl butyral resins include reaction products of polyvinyl alcohols with butyl aldehyde, and reaction products in which crosslinking between the molecules has been carried out with monobutyral bonds.

Examples of phenolic resins include resins obtained using organic compounds belonging to the phenols, such as phenol or cresol.

Examples of amino resins include urea resins, melamine resins and guanamine resins.

Examples of oxazoline resins include bisoxazoline compounds and terminal oxazoline group-containing compounds obtained by reacting 2 chemical equivalents of oxazoline groups on a bisoxazoline compound with 1 chemical equivalent of carboxyl groups on a polybasic carboxylic acid. Alternatively, the oxazoline compound may be a polymerized compound having two or more oxazoline groups per molecule that is obtained from a polymer prepared by addition polymerization or the like without ring opening of the oxazoline rings. Additional examples include copolymers of an addition-polymerizable oxazoline compound with a copolymerizable monomer that does not react with oxazoline groups.

Examples of carbodiimide resins include resins having at least one carbodiimide group obtained using one, two or more isocyanate compound as the starting material.

Of these, polymer particle A is more preferably made of a styrene resin, (meth)acrylic resin, polyolefin resin, vinyl ester resin, poly-N-vinyl compound resin or polydiene resin.

Polymer particle A is most preferably made up of a (co)polymer composed of at least one monomer selected from among styrenes, (meth)acrylic acids, (meth)acrylic esters, vinyl esters, N-vinyl compounds, olefins, fluorinated olefins and conjugated dienes.

Illustrative examples include polystyrene, styrene-(meth)acrylic acid copolymers, styrene-(meth)acrylic ester copolymers, poly(meth)acrylic acids, polymethyl (meth)acrylate, polyethyl (meth)acrylate, polybutyl (meth)acrylate, (meth)acrylic acid-methyl (meth)acrylate copolymers, (meth)acrylic ester copolymers, polyvinyl acetate, poly-N-vinylpyrrole, poly-N-vinylcarbazole, poly-N-vinylindole, poly-N-vinylpyrrolidone, polyethylene, polypropylene, polyvinyl fluoride, polytetrafluoroethylene, polybutadiene, polyisoprene, and copolymers thereof.

Of these, (co)polymers containing, as essential units, repeating units obtained from at least one monomer selected from among styrenes, (meth)acrylic acids and (meth)acrylic esters are preferred. Polystyrene, styrene-(meth)acrylic acid copolymers, styrene-(meth)acrylic ester copolymers, poly(meth)acrylic acids, polymethyl (meth)acrylate, polyethyl (meth)acrylate, polybutyl (meth)acrylate, (meth)acrylic acid-methyl (meth)acrylate copolymers and (meth)acrylic ester copolymers are especially preferred.

Polymer particle A may be a mixture of two or more types, provided it satisfies above conditions (1) to (3).

The UV scattering agent of the invention further includes at least one type of particle B which has (4) a shape differing from that of polymer particle A and a volume mean particle size (MV_{B}) that satisfies the condition 1/5 × D_{AV} ≤ MV_{B} ≤ L_{AV}.

The differing shape mentioned here includes also elliptical or needle-like particles of a shape or size differing from that of polymer particle A, although for ease of orientation and to stably obtain optical properties such as a UV scattering effect, light scattering ability in the visible light region and light reflectivity, any shape that differs from an elliptical or needle-like shape is preferred. Examples of such shapes include spherical, substantially spherical, platy, flaky, pulverized, rod-like, polyhedral, rough, and block-like shapes. As used herein, "substantially spherical" refers to an elliptical shape having an aspect ratio of less than 2.

According to studies by the inventors, polymer particle A has, owing to its shape, a higher UV scattering effect than conventional spherical or substantially spherical particles of the same composition. However, owing to its flowability during the production of molded articles and compositions and during formulation, polymer particle A tends to orient itself in the direction of flow, in which case optical properties such as the UV scattering effect, the light-scattering ability in the visible light region and the light reflectivity may not be stably obtained. In such cases, the addition of a specific particle B imparts a sterically hindering effect, thereby enabling the anisotropic characteristics to be stably controlled. It is therefore possible with particle B to maintain a stable UV scattering effect without adversely affecting the polymer particle A characteristics.

The volume mean particle size (MV_{B}) of particle B preferably satisfies the condition 1/3 × D_{AV} ≤ MV_{B} ≤ 0.8 × L_{AV}, more preferably satisfies the condition 1/2 × D_{AV} ≤ MV_{B} ≤ 0.6 × L_{AV}, and even more preferably satisfies the condition D_{AV} ≤ MV_{B} ≤ 1/2 × L_{AV}. When MV_{B} is lower than 1/5 × D_{AV}, there is a lack of stability in the steric hindrance. On the other hand, when MV_{B} is greater than L_{AV}, the UV scattering effect more readily depends on the volume mean particle size, which may lower the UV scattering effect.

Particle B may be an inorganic particle or a polymer particle. In order for such characteristics of polymer particle A as its optical properties, gloss, permeability, weight-reducing ability and tactile qualities to be fully manifested, it is preferable for particle B to be a polymer particle. In this case, from the standpoint of production efficiency, it is preferable for the polymer making up particle B to have the same ingredients as polymer particle A or to at least include the same ingredients. When taking into account also improvement in the ability to scatter light in the visible wavelength region, a composition in which polymer particles A and B have differing refractive indices is effective.

Particle B, so long as it satisfies condition (4), may be a mixture of two or more types. Depending on the intended use, it may even be a mixture of polymer particles and inorganic particles.

To further improve the UV scattering effects and the visible light scattering effects, it is preferable for at least one of polymer particle A and particle B to have at least any one of the following characteristics: fine irregularities (roughness) on the particle surface, porosity, or a relatively large specific surface area.

Also, at least one of polymer particle A and particle B may be a composite particle having a core-shell structure, or a composite particle obtained by the physical or chemical addition of other fine particles.

Examples of methods for producing composite particles include (1) incorporating other fine particles at the time of parent particle production, (2) using the polarity of ionic functional groups present at the surface of the parent particles following parent particle production to add other fine particles, and (3) chemical methods such as addition polymerization, polycondensation, addition condensation or seed polymerization.

As used herein, "other fine particles" refers to organic or inorganic particles which are smaller than polymer particle A and particle B serving as the parent particles. The preferred particle size of the other fine particles varies according to the size of the parent particles, but is generally in the range of about 0.005 to 50 µm.

Such organic particles are exemplified by particles composed of polymerizable monomers that may be used in the production of polymer particles, curable particles and organic pigments.

Such inorganic particles are exemplified by metals, metal oxides, hydrated metal oxides and inorganic pigments such as copper powder, iron powder, gold powder, aluminum oxide, titanium oxide, zinc oxide, silicon oxide, tin oxide, copper oxide, iron oxide, magnesium oxide, manganese oxide, calcium carbonate, magnesium hydroxide and aluminum hydroxide.

These fine particles are exemplified by commercial products that may be used directly as is or may be used following surface modification with a surface treatment agent such as a coupling agent.

Particularly in cases where polymer particle A and particle B are used in optical applications, metal oxide fine particles having a particle size of 0.005 to 10 µm, especially titanium oxide, zinc oxide or silicon oxide, may be added to control the refractive index and improve the UV scattering effect. These may be used singly or two or more may be used in combination.

These metal oxide fine particles can be incorporated by, during production of the polymer particles, adding from 0.1 to 50 wt% of the fine particles, based on the total amount of the polymerization ingredients, and physically/chemically adsorbing or bonding the fine particles within the resulting polymer particles. By thus including a suitable amount of inorganic particles in or coating a suitable amount of inorganic particles on the polymer fine particles to form a composite, it is possible to further enhance the UV scattering effects while retaining gloss.

For use in specimens of biological/pharmaceutical materials, such as cosmetics or pharmaceutical preparations, efficacy as a drug can be conferred by physically, mechanically or chemically bonding an active material as appropriate. Known techniques may be employed for such physical, mechanical or chemical bonding. Thus, for ingredients that are to be dissolved, use can be made of techniques that absorb/adsorb the ingredient at the particle interior or on a surface layer; use can be made of coloring techniques with dyes or the like; and use can be made of known chemical bonding techniques that chemically bond reactive groups present at the particle interior or on a surface layer with reactive groups on the active material to effect adsorption.

Here, "reactive groups" refers to polymerizable unsaturated bond-containing groups such as α,β-unsaturated carbonyl group, α,β-unsaturated nitrile groups, halovinyl groups, halovinylidene groups, aromatic vinyl groups, heterocyclic vinyl groups, conjugated dienes and carboxylic acid vinyl esters; and also carboxyl, carbonyl, epoxy, isocyanate, hydroxyl, amido, cyano, amino, epoxy, chloromethyl, glycidyl ether, lithio, ester, formyl, nitrile, nitro, carbodiimide and oxazoline groups.

In the UV scattering agent of the invention including both polymer particle A and particle B, the mixing ratio (weight ratio) therebetween is preferably between 99:1 and 10:90, more preferably between 98:2 and 30:70, even more preferably between 97:3 and 50:50, and most preferably between 95:5 and 80:20. When the mixing ratio of particle B exceeds 90 wt%, the UV scattering effect may be accompanied at the same time by a large decrease in the optical characteristics of the polymer particle A in the visible light region. On the other hand, when the mixing ratio of particle B is less than 1 wt%, the UV scattering effect may be accompanied at the same time by problems with the stability of the optical characteristics of polymer particle A in the visible light region.

### [Polymer Particle Production Method]

The method of producing polymer particles is not particularly limited, provided it is a method capable of obtaining particles of the shape described above. For example, when particle B is a polymer particle, this may be obtained by grinding, solution polymerization or the like. Polymer particle A can be obtained by the methods described in, for example, Patent Documents 1 to 5. Production by solution polymerization, which enables easy control of the particle size obtained using common unsaturated double bond-containing monomers, is preferred.

Solution polymerization is exemplified by (1) emulsion or suspension polymerization carried out in an aqueous solution, (2) dispersion polymerization carried out in the presence of a dispersant in water, an organic solvent, or a mixed solvent of water and an organic solvent, and (3) a combination of above method (1) or (2) with seed polymerization. The use of solution polymerization that is a combination of (1) and (2) is especially preferred for the production of polymer particle A.

According to studies by the inventors, when particle B is a polymer particle and has the same ingredients as polymer particle A, suitable production of the desired mixed particles can be easily carried out by varying the types and relative amounts of the monomers and solvents, and also, where necessary, the relative amounts of dispersants and emulsifiers used.

The production methods described in Patent Documents 4 and 5 are suitable for conferring the polymer particles with the following characteristics described above: fine irregularities (roughness) on the fine particle surface, porosity, and a large specific surface area.

Examples of the polymerizable monomer serving as the starting material for the polymer particle used in this invention include:
(i) styrene compounds such as styrene, o-methylstyrene, m-methylstyrene, p-methylstyrene, α-methylstyrene, o-ethylstyrene, m-ethylstyrene, p-ethylstyrene, 2,4-dimethylstyrene, p-n-butylstyrene, p-t-butylstyrene, p-n-hexylstyrene, p-n-octylstyrene, p-n-nonylstyrene, p-n-decylstyrene, p-n-dodecylstyrene, p-methoxystyrene, p-phenylstyrene, p-chlorostyrene and 3,4-dichlorostyrene;
(ii) (meth)acrylic acids;
(iii) hydrocarbon group-containing (meth)acrylic monomers such as methyl (meth)acrylate, ethyl (meth)acrylate, propyl (meth)acrylate, n-butyl (meth)acrylate, isobutyl (meth)acrylate, pentyl (meth)acrylate, hexyl (meth)acrylate, 2-ethylhexyl (meth)acrylate, octyl (meth)acrylate, nonyl (meth)acrylate, decyl (meth)acrylate, dodecyl (meth)acrylate, lauryl (meth)acrylate, stearyl (meth)acrylate, cyclohexyl (meth)acrylate, isobornyl (meth)acrylate, phenyl (meth)acrylate, toluyl (meth)acrylate and benzyl (meth)acrylate; fluorine-containing (meth)acrylic monomers such as 2,2,2-trifluoroethyl (meth)acrylate, 3,3,3-trifluoropropyl (meth)acrylate, 2-(perfluoroethyl)ethyl (meth)acrylate, 2-perfluoroethyl-2-perfluorobutylethyl (meth)acrylate, 2-perfluoroethyl (meth)acrylate, tetrafluoropropyl (meth)acrylate, perfluoromethyl (meth)acrylate, 1,1,1,3,3,3-hexafluoropropan-2-yl (meth)acrylate, 2-perfluoromethyl-2-perfluoroethylmethyl (meth)acrylate, 2-(perfluorohexyl)ethyl meth)acrylate, 2-(perfluorodecyl)ethyl (meth)acrylate and 2-(perfluorohexadecyl)ethyl (meth)acrylate; hydroxyl group-containing (meth)acrylic monomers such as 2-hydroxyethyl (meth)acrylate, 2-hydroxypropyl (meth)acrylate, 3-hydroxypropyl (meth)acrylate and 4-hydroxybutyl (meth)acrylate; epoxy group-containing (meth)acrylic monomers such as glycidyl (meth)acrylate, (β-methyl)glycidyl (meth)acrylate and 3,4-epoxycyclohexyl (meth)acrylate; amino group-containing (meth)acrylic monomers such as 2-aminoethyl (meth)acrylate, N-propylaminoethyl (meth)acrylate, N-ethylaminopropyl (meth)acrylate, N-phenylaminoethyl (meth)acrylate and N-cyclohexylaminoethyl (meth)acrylate; silicon-containing (meth)acrylic monomers such as 3-(meth)acryloyloxypropyltrimethoxysilane and 3-(meth)acryloyloxypropyldimethoxymethylsilane; alkoxy group-containing (meth)acrylic monomers such as (poly)ethylene glycol mono(meth)acrylate, 2-methoxyethyl (meth)acrylate and 3-methoxybutyl (meth)acrylate; (poly)alkylene glycol (meth)acrylic monomers such as (poly)propylene glycol mono(meth)acrylate; alkoxy(poly)alkylene glycol (meth)acrylic monomers such as methoxy(poly)ethylene glycol mono(meth)acrylate and methoxy(poly)propylene glycol mono(meth)acrylate; mercapto group-containing (meth)acrylic monomers such as 2-mercaptoethyl (meth)acrylate and 2-mercapto-1-carboxyethyl (meth)acrylate; and (meth)acrylic esters such as 2-chloroethyl (meth)acrylate and methyl α-chloro(meth)acrylate;
(iv) vinyl esters such as vinyl acetate, vinyl propionate, vinyl benzoate, vinyl butyrate, vinyl formate, vinyl valerate and vinyl pivalate;
(v) N-vinyl compounds such as N-vinylpyrrole, N-vinylcarbazole, N-vinylindole and N-vinylpyrrolidone;
(vi) olefins such as ethylene and propylene;
(vii) fluorinated olefins such as vinyl fluoride, vinylidene fluoride, tetrafluoroethylene and hexafluoropropylene; and
(viii) conjugated dienes such as butadiene and isoprene. These may be used singly or two or more may be used in combination.

Of these, the use of styrenes, (meth)acrylic acids, (meth)acrylic esters and vinyl esters as the polymerizable monomer is preferred. By using these, polymer particles having the above-described shape can be easily and inexpensively obtained.

Aside from the above polymerizable monomers, use can be made of unsaturated monomers having a reactive functional group such as a hydrophilic functional group or an active hydrogen group. The reactive functional group is exemplified by amino, carboxyl, hydroxyl, thiol, carbonyl, ether, cyano, amide, alkylene oxide, epoxy and ionic functional groups. The unsaturated monomer may have only one type, or a mixture of two or more types, of the foregoing functional groups. By introducing reactive functional groups such as these hydrophilic functional groups or active hydrogen groups to the interior of the particle or onto the surface layer of the particle, not only can functionality such as hydrophilicity and oil resistance be enhanced, it is also possible to use these reactive functional groups as auxiliary functional groups which impart various types of functionality, such as forming a composite of the inorganic particles with other polymer fine particles, forming a crosslinked structure due to reactions between the functional groups, surface treatment and surface modification due to the bonding of reactive compounds, and the furnishing of active substances.

Examples of unsaturated monomers having such reactive functional groups include those mentioned below. In the description that follows, "Cₙ" refers to the number of carbon atoms.

### (1) Amino Group-Containing Monomers

Examples include allylamine derivatives such as allylamine and N-methylallylamine, amino group-containing styrene derivatives such as p-aminostyrene, and triazine derivatives such as 2-vinyl-4,6-diamino-S-triazine. Of these, compounds having a primary or secondary amino group are preferred.

### (2) Carboxyl Group-containing Monomers

Examples include unsaturated carboxylic acids such as crotonic acid, cinnamic acid, itaconic acid, maleic acid and fumaric acid, mono(C₁-C₈ alkyl) esters of itaconic acid such as mono-butyl itaconate, mono(C₁-C₈ alkyl) esters of maleic acid such as mono-butyl maleate, vinyl group-containing aromatic carboxylic acids such as vinylbenzoic acid, and salts of these.

### (3) Hydroxyl Group-Containing Monomers

Examples include hydroxyalkyl vinyl ether monomers such as hydroxyethyl vinyl ether and hydroxybutyl vinyl ether, and hydroxyl group-containing allyl monomers such as allyl alcohol and 2-hydroxyethyl allyl ether.

### (4) Thiol (Mercapto) Group-Containing Monomers

Examples include N-(2-mercaptoethyl)acrylamide, N-(2-mercapto-1-carboxyethyl)acrylamide, N-(2-mercaptoethyl)methacrylamide, N-(4-mercaptophenyl)acrylamide, N-(7-mercaptonaphthyl)acrylamide and mono(2-mercaptoethylamide) maleate.

### (5) Carbonyl Group-Containing Monomers

Examples include vinyl ketones such as vinyl methyl ketone, vinyl hexyl ketone and methyl isopropenyl ketone.

### (6) Ether Group-Containing Monomers

Examples include vinyl ether monomers such as vinyl methyl ether, vinyl ethyl ether and vinyl isobutyl ether.

### (7) Cyano Group-Containing Monomers

Examples include acrylonitrile, methacrylonitrile, hexenenitrile, 4-pentenenitrile and p-cyanostyrene.

### (8) Amide Group-Containing Monomers

Examples include (meth)acrylamide, α-ethyl (meth)acrylamide, N-methyl (meth)acrylamide, N-butoxymethyl (meth)acrylamide, diacetone (meth)acrylamide, N,N-dimethyl (meth)acrylamide, N,N-diethyl (meth)acrylamide, N,N-dimethyl-p-styrenesulfonamide, N,N-dimethylaminoethyl (meth)acrylamide, N,N-diethylaminoethyl (meth)acrylamide, N,N-dimethylaminopropyl (meth)acrylamide and N,N-diethylaminopropyl (meth)acrylamide.

### (9) Epoxy Group-Containing Monomers

Examples include allyl glycidyl ether, 3,4-epoxyvinylcyclohexane, di(β-methyl)glycidyl maleate and di(β-methyl)glycidyl fumarate.

### (10) Ionic Functional Group-Containing Monomers

The ionic functional groups may be either anionic functional groups or cationic functional groups. Examples of anionic functional groups include carboxyl groups, sulfonic acid groups, phosphoric acid groups, phenolic hydroxyl groups and salts thereof. Examples of cationic functional groups include amino groups, imidazole groups, pyridine groups, amidino groups, and salts thereof.

Anionic functional groups are especially preferred on account of the many general-purpose products and wealth of types available, and also because they make it possible to efficiently control the size, shape and other properties of elliptical or needle-like polymer particles. Of these, the use of one or more type of functional group selected from among carboxyl groups, sulfonic acid groups, phosphoric acid groups and derivatives thereof is particularly preferable because such groups are easy to introduce into molecules and have an excellent stability and safety.

Compounds capable of serving as counterions to such ionic functional groups are exemplified by, for anionic functional groups: metal cations, ammonium cations, pyridinium cations and phosphonium cations; and for cationic functional groups: halide ions such as chloride, bromide and iodide ions.

When an anionic functional group is used, for reasons having to do with production costs, the wealth of types and the ability to efficiently control such characteristics of elliptical or needle-like particles as their accuracy, size and shape, it is most preferable for the counterion to be a metal cation.

Examples of metal cations include alkali metal cations such as lithium, sodium, rubidium and cesium; alkaline earth metal cations such as magnesium, calcium, strontium and barium; other non-transition metal cations such as aluminum; and transition metal cations such as zinc, copper, manganese, nickel, cobalt, iron and chromium.

Monomers having an anionic functional group are exemplified by monocarboxylic acid monomers, dicarboxylic acid monomers, sulfonic acid monomers, sulfate ester monomers, phenolic hydroxyl group-containing monomers and phosphoric acid monomers.

Examples of monocarboxylic acid monomers include (meth)acrylic acid, crotonic acid, cinnamic acid, mono(C₁-C₈ alkyl) esters of maleic acid, mono(C₁-C₈ alkyl) esters of itaconic acid, vinylbenzoic acid, and salts thereof.

Examples of dicarboxylic acid monomers include maleic acid and its anhydride, α-methylmaleic acid and its anhydride, α-phenylmaleic acid and its anhydride, fumaric acid, itaconic acid, and salts thereof.

Examples of sulfonic acid monomers include alkenesulfonic acids such as ethylenesulfonic acid, vinylsulfonic acid and (meth)allylsulfonic acid; aromatic (styrene) sulfonic acids such as styrenesulfonic acid and α-methylstyrenesulfonic acid; C₁-C₁₀ alkyl (meth)allylsulfosuccinates; sulfo-C₂-C₆ alkyl (meth)acrylates such as sulfopropyl (meth)acrylate; and sulfonic acid group-containing unsaturated esters such as methylvinylsulfonate, 2-hydroxy-3-(meth)acryloxypropylsulfonic acid, 2-(meth)acryloylamino-2,2-dimethylethanesulfonic acid, 3-(meth)acryloyloxyethanesulfonic acid, 3-(meth)acryloyloxy-2-hydroxypropanesulfonic acid, 2-(meth)acrylamido-2-methylpropanesulfonic acid and 3-(meth)acrylamido-2-hydroxypropanesulfonic acid, as well as salts thereof.

Examples of sulfate ester monomers include the sulfate esters of (meth)acryloyl polyoxyalkylenes (degree of polymerization, 2 to 15), such as the sulfate ester of polyoxypropylene monomethacrylate, and salts thereof.

Examples of phenolic hydroxyl group-containing monomers include hydroxystyrene, bisphenol A monoallyl ether, bisphenol A mono(meth)acrylate ester, and salts thereof.

Examples of phosphoric acid monomers include (meth)acrylic acid hydroxyalkyl phosphate monoesters such as 2-hydroxyethyl (meth)acryloyl phosphate and phenyl-2-acryloyloxy ethyl phosphate; and vinylphosphoric acid.

Examples of the salts in these cases include alkali metal salts such as sodium salts and potassium salts, amine salts such as triethanolamine, and quaternary ammonium salts such as tetra-C₄-C₁₈ alkylammonium salts.

Monomers having a cationic functional group are exemplified by primary amino group-containing monomers, secondary amino group-containing monomers, tertiary amino group-containing monomers, quaternary ammonium salt group-containing monomers, heterocycle-containing monomers, phosphonium group-containing monomers, sulfonium group-containing monomers and sulfonic acid group-containing polymerizable unsaturated monomers.

Examples of primary amino group-containing monomers include C₃-C₆ alkenylamines such as allylamine and crotylamine; amino-C₂-C₆ alkyl (meth)acrylates such as aminoethyl (meth)acrylate; monomers having an aromatic ring and a primary amino group, such as vinylaniline and p-aminostyrene; ethylenediamine, and polyalkylene polyamines.

Examples of secondary amino group-containing monomers include C₁-C₆ alkylamino-C₂-C₆ alkyl (meth)acrylates such as t-butylaminoethyl (meth)acrylate and methylaminoethyl (meth)acrylate; C₆-C₁₂ dialkenylamines such as di(meth)allylamine; ethyleneimine; and diallylamine.

Examples of tertiary amino group-containing monomers include di (C₁-C₄ alkylamino-C₂-C₆ alkyl) (meth) acrylates such as N,N-dimethylaminoethyl (meth)acrylate, N,N-diethylaminoethyl (meth)acrylate, N,N-dimethylaminopropyl (meth)acrylate, N,N-diethylaminopropyl (meth)acrylate, N,N-dibutylaminoethyl (meth)acrylate, N-t-butylaminoethyl (meth)acrylate and N,N-dimethylaminobutyl (meth)acrylate; di (C₁-C₄ alkylamino-C₂-C₆ alkyl) (meth) acrylamides such as N,N-dimethylaminoethyl (meth)acrylamide and N,N-dimethylaminopropyl (meth)acrylamide; and monomers having an aromatic ring and a tertiary amino group, such as N,N-dimethylaminostyrene.

Examples of quaternary ammonium salt group-containing monomers include tertiary amines that have been quaternized using a quaternizing agent such as a C₁-C₁₂ alkyl chloride, a dialkylsulfuric acid, a dialkyl carbonate or benzyl chloride.

Specific examples include alkyl (meth)acrylate-type quaternary ammonium salts such as [2-((meth)acryloyloxy)ethyl]trimethylammonium chloride, [2-((meth)acryloyloxy)ethyl]trimethylammonium bromide, [(meth)acryloyloxy)ethyl]triethylammonium chloride, [(meth)acryloyloxy)ethyl]dimethylbenzylammonium chloride and [(meth)acryloyloxy)ethyl]methylmorpholinoammonium chloride; alkyl (meth)acrylamide-type quaternary ammonium salts such as [(meth)acryloylamino)ethyl]trimethylammonium chloride, [(meth))acryloylamino)ethyl]trimethylammonium bromide, [(meth)acryloylamino)ethyl]triethylammonium chloride and [(meth)acryloylamino)ethyl]dimethylbenzylammonium chloride; and other quaternary ammonium salt group-containing monomers such as dimethyldiallylammonium methyl sulfate, trimethylvinylphenylammonium chloride, tetrabutylammonium (meth)acrylate, trimethylbenzylammonium (meth)acrylate and 2-(methacryloyloxy)ethyltrimethylammonium dimethylphosphate.

Examples of heterocycle-containing monomers include N-vinylcarbazole, N-vinylimidazole, N-vinyl-2,3-dimethylimidazoline, N-methyl-2-vinylimidazoline, 2-vinylpyridine, 4-vinylpyridine, N-methylvinylpyridine and oxyethyl-1-methylenepyridine.

An example of a phosphonium group-containing monomers is glycidyl tributylphosphone.

Examples of sulfonium group-containing monomers include 2-acryloxyethyldimethyl sulfone and glycidylmethylsulfonium.

Examples of sulfonic acid group-containing polymerizable unsaturated monomers include (meth)acrylamidoalkanesulfonic acids such as 2-acrylamido-2-methylpropanesulfonic acid, and sulfoalkyl (meth)acrylates such as 2-sulfoethyl (meth)acrylate.

The above-mentioned cationic functional group-containing monomers may be used in the form of inorganic acid salts such as hydrochlorides and phosphates, or in the form of organic acid salts such as formates and acetates.

The reactive functional group-containing unsaturated monomers mentioned above may be used singly or two or more may be used in combination.

Of the above reactive functional group-containing unsaturated monomers, a monomer having a hydroxyl, carboxyl, amino, amide, alkylene oxide or ionic functional group is preferred, and a monomer having a hydroxyl, carboxyl, ethylene oxide or ionic functional group is more preferred. By using these functional groups, the hydrophilic properties are strengthened and repulsion between the particles obtained in solution becomes stronger, increasing the stability of the dispersion and making it possible to enhance even further the monodispersibility. This in turn makes it possible to reduce deterioration in particle size accuracy due to sticking and agglomeration, and also enables polymer particles endowed with excellent chemical resistance, reactivity, solution dispersibility, powder dispersibility and mechanical properties to be obtained.

Moreover, it is preferable for the unsaturated monomer having a reactive functional group to be a water-soluble compound. By using a water-soluble monomer, the monodispersibility can be still further enhanced, in addition to which the polymer particles obtained can be easily dispersed in water or an aqueous medium.

Also, for heat-resistant or chemical-resistant applications of the resulting particles, a suitable amount of from 0.01 to 80 wt% of a crosslinking agent, based on the total weight of the polymerization ingredients, may be included at the time of the polymerization reaction. Examples of the crosslinking agent include aromatic divinyl compound such as divinylbenzene and divinylnaphthalene; and other compounds such as ethylene glycol diacrylate, ethylene glycol dimethacrylate, triethylene glycol dimethacrylate, tetraethylene glycol dimethacrylate, 1,3-butylene glycol dimethacrylate, trimethylolpropane triacrylate, trimethylolpropane trimethacrylate, 1,4-butanediol diacrylate, neopentyl glycol diacrylate, 1,6-hexanediol diacrylate, pentaerythritol triacrylate, pentaerythritol tetraacrylate, pentaerythritol dimethacrylate, pentaerythritol tetramethacrylate, glycerol acryloxy dimethacrylate, N,N-divinylaniline, divinyl ether, divinylsulfide and divinylsulfone. These may be used singly or two or more may be used in combination.

Any of various known polymerization initiators may be used as the initiator when carrying out the polymerization reaction. The initiator is exemplified by various oil-soluble, water-soluble or ionic polymerization initiators, including peroxides such as benzoyl peroxide, cumene hydroperoxide, t-butyl hydroperoxide, sodium persulfate and ammonium persulfate; and azo compounds such as azobisisobutyronitrile, azobismethylbutyronitrile, azobisisovaleronitrile, 2,2'-azobis(2-amidinopropane) dihydrochloride, 2,2'-azobis(N,N'-dimethyleneisobutylamidine) dihydrochloride and disodium 2,2'-azobis-2-cyanopropane-1-sulfonate. These polymerization initiators may be used singly or two or more may be used in combination. The amount of radical polymerization initiator included is generally from 0.1 to 50 parts by weight per 100 parts by weight of the starting monomer.

The solvent used in synthesis is not particularly limited. Any solvent that is suitable for the starting materials to be used may be selected from among ordinary solvents.

Examples of solvents that may be used include water and the following hydrophilic organic solvents: methanol, ethanol, 1-propanol, 2-propanol, ethylene glycol, propylene glycol, methyl cellosolve, ethyl cellosolve, propyl cellosolve, methyl cellosolve acetate, ethyl cellosolve acetate, methyl carbitol, ethyl carbitol, butyl carbitol, ethyl carbitol acetate, acetone, tetrahydrofuran, dimethylformamide, N-methyl-2-pyrrolidone and acetonitrile. These may be used singly or two or more may be used in admixture.

As used herein, "hydrophilic organic solvent" refers to a solvent which, as a mixture with water, maintains a uniform appearance. "Hydrophobic organic solvent" refers to a solvent for which, when the solvent is calmly stirred with the same volume of pure water at one atmosphere and a temperature of 20°C, the resulting mixed liquid is unable to maintain a uniform appearance once the flow of liquid has subsided.

A hydrophobic organic solvent may also be used. Examples include higher alcohols such as 1-butanol, 2-butanol, isobutanol, tert-butanol, 1-pentanol, 2-pentanol, 3-pentanol, 2-methyl-1-butanol, isopentyl alcohol, tert-pentyl alcohol, 1-hexanol, 2-methyl-1-pentanol, 4-methyl-2-pentanol, 2-ethylbutanol, 1-heptanol, 2-heptanol, 3-heptanol, 2-octanol, 2-ethyl-1-hexanol, benzyl alcohol and cyclohexanol; ether alcohols such as butyl cellosolve; ketones such as methyl ethyl ketone, methyl isobutyl ketone and cyclohexanone; esters such as ethyl acetate, butyl acetate, ethyl propionate and butyl carbitol acetate; aliphatic or aromatic hydrocarbons such as pentane, 2-methylbutane, n-hexane, cyclohexane, 2-methylpentane, 2,2-dimethylbutane, 2,3-dimethylbutane, heptane, n-octane, isooctane, 2,2,3-trimethylpentane, decane, nonane, cyclopentane, methylcyclopentane, methylcyclohexane, ethylcyclohexane, p-menthane, dicyclohexyl, benzene, toluene, xylene and ethylbenzene; and halogenated hydrocarbons such as carbon tetrachloride, trichloroethylene, chlorobenzene and tetrabromoethane. These may be used singly or two or more may be used in admixture.

In order to impart the above-mentioned characteristics of fine surface irregularities, porosity and a large specific surface area to at least one of polymer particle A and B, it is preferable to use a mixed solvent of water and a hydrophilic organic solvent or such a mixed solvent in combination with a hydrophobic organic solvent. In this way, the particle surface and interior can be suitably modified.

The above solvents may be mixed in any proportions, the mixing proportions being suitably adjusted according to the monomers to be used. For example, the weight ratio of water to organic solvents other than water may be set in the range of 1:99 to 99:1. However, for the target fuzzy state to be easily achieved, and also to improve the (co)polymerizability and more efficiently obtain particles having a smaller particle size and a high aspect ratio, a ratio of 10:90 to 80:20, and especially 30:70 to 70:30, is preferred. Here, "fuzzy state" refers to, by the optional selection of a mixed solvent, forming a state having both areas where monomer is dissolved and areas where monomer is dispersed; that is, a state having at least both emulsified areas and dissolved areas. By thus forming a fuzzy state, it is possible, for example, to efficiently modify the surface, reduce the size and adjust the aspect ratio of the particles obtained following polymerization (Patent Document 5).

When a mixed solvent of a hydrophilic organic solvent and a hydrophobic organic solvent is used as the organic solvent, for the same reasons as given above, the weight ratio of hydrophilic organic solvent to hydrophobic organic solvent is set in the range of preferably 10:90 to 90:10, more preferably 80:20 to 20:80, and most preferably 70:30 to 30:70.

In this invention, by carrying out such adjustments in the solvent composition, the particle size and aspect ratio, the size of fine surface irregularities and the porosity of the polymer particles can be controlled, enabling the UV scattering effects to be improved and a good balance to be achieved in performance attributes such as the optical characteristics, water absorbency and oil absorbency.

The content of starting monomer in the reaction mixture is set to preferably 1 to 80 wt%, more preferably 5 to 50 wt%, and even more preferably 10 to 30 wt%, based on the overall reaction mixture. When the content of starting monomer exceeds 80 wt%, obtaining in a high yield polymer particles having the above properties in a monodispersed state is difficult. On the other hand, when the content is less than 1 wt%, the reaction takes a long time to reach completion, which is impractical from an industrial point of view.

The reaction temperature during polymerization varies depending on the type of solvent used and so cannot be strictly specified, although it is typically about 10 to 200°C, preferably 30 to 130°C, and more preferably 40 to 90°C.

The reaction time is not particularly limited, provided it is the time required for the target reaction to go substantially to completion. The reaction time is largely governed by such factors as the type and content of the monomer, the viscosity and concentration of the solution, and the target particle size. For example, at 40 to 90°C, the reaction time may be from 1 to 72 hours, and is preferably about 2 to 24 hours.

When producing the polymer particles used in this invention, depending on the polymerization method, other additives such as (polymer) dispersants, stabilizers and emulsifying agents (surfactants) may be included in a suitable amount of 0.01 to 50 wt%, based on the starting monomer.

The dispersants and stabilizers are exemplified by various types of hydrophobic or hydrophilic dispersants and stabilizers, including polystyrene derivatives such as polyhydroxystyrene, polystyrenesulfonic acid, hydroxystyrene-(meth)acrylic ester copolymers, styrene-(meth)acrylic ester copolymers and styrene-hydroxystyrene-(meth)acrylic ester copolymers; poly(meth)acrylic acid derivatives such as poly(meth)acrylic acid, poly(meth)acrylamide, polyacrylonitrile, polyethyl (meth)acrylate and polybutyl (meth)acrylate; poly(vinyl alkyl ether) derivatives such as poly(methyl vinyl ether), poly(ethyl vinyl ether), poly(butyl vinyl ether) and poly(isobutyl vinyl ether); cellulose and cellulose derivatives such as methyl cellulose, cellulose acetate, cellulose nitrate, hydroxymethyl cellulose, hydroxyethyl cellulose, hydroxypropyl cellulose and carboxymethyl cellulose; polyvinyl acetate derivatives such as polyvinyl alcohol, polyvinyl butyral, polyvinyl formal and polyvinyl acetate; nitrogen-containing polymer derivatives such as polyvinyl pyridine, polyvinyl pyrrolidone, polyethyleneimine and poly-2-methyl-2-oxazoline; and polyvinyl halide derivatives such as polyvinyl chloride and polyvinylidene chloride. These may be used singly or two or more may be used in combination.

The emulsifying agents (surfactants) are exemplified by anionic emulsifying agents, including alkyl sulfates such as sodium dodecylsulfate, alkylbenzene sulfonates such as sodium dodecylbenzene sulfonate, alkylnaphthalene sulfonates, fatty acid salts, alkyl phosphates and alkyl sulfosuccinates; cationic emulsifying agents such as alkylamines, quaternary ammonium salts, alkyl betaines and amine oxides; and nonionic emulsifying agents such as polyoxyethylene alkyl ethers, polyoxyethylene alkylallyl ethers, polyoxyethylene alkylphenyl ethers, sorbitan fatty acid esters, glycerol fatty acid esters and polyoxyethylene fatty acid esters. These may be used singly or two or more may be used in combination.

Depending on the intended use of the resulting particles, a catalyst (reaction promoter) may be included in the polymerization reaction. The amount of catalyst included may be a suitable amount that does not adversely affect the particle properties, such as from 0.01 to 20 wt%, based on the combined weight of the polymerization ingredients.

The catalyst is not subject to any particular limitation, provided it is a positive catalyst. Any suitable known catalyst may be selected and used. Specific examples include tertiary amines such as benzyldimethylamine, triethylamine, tributylamine, pyridine and triphenylamine; quaternary ammonium compounds such as triethylbenzylammonium chloride and tetramethylammonium chloride; phosphines such as triphenylphosphine and tricyclophosphine; phosphonium compounds such as benzyltrimethylphosphonium chloride; imidazole compounds such as 2-methylimidazole and 2-methyl-4-ethylimidazole; alkali metal hydroxides such as potassium hydroxide, sodium hydroxide and lithium hydroxide; alkali metal carbonates such as sodium carbonate and lithium carbonate; alkali metal salts of organic acids; and halides or complex salts thereof which exhibit Lewis acid properties, such as boron trichloride, boron trifluoride, tin tetrachloride and titanium tetrachloride. These may be used singly or two or more may be used in combination.

In addition, to adjust such characteristics as the size, shape and quality of the resulting elliptical or needle-like polymer particles, compounds that are capable of dissolving in water or another polar solvent, that undergo electrolytic dissociation into cations and anions, and that exhibit electrical conductivity in solution may also be added at the time of the polymerization reaction.

Examples of such compounds include salts, inorganic acids, inorganic bases, organic acids, organic bases and ionic liquids. The amount of addition may be set to a suitable amount which does not adversely affect the particle properties, such as from 0.01 to 80 wt%, based on the combined weight of the polymerization ingredients.

### [Applications of UV Scattering Agent]

The UV scattering agent of the invention is suitable as a UV-cutting additive.

Compositions containing the UV scattering agent of the invention exhibit a number of advantageous effects; that is, they have excellent manufacturability when mixed into a polymer or dispersed in a medium, do not give rise to bleed-out with long-term use, have an excellent UV scattering performance and, moreover, by maintaining the scattering performance over a long period of time, have an excellent light resistance (UV fastness). Also, they do not have a structure that irritates the skin, and thus are easy to handle.

The UV scattering agent of the invention has excellent light resistance, and thus can be used in polymer molded or shaped articles such as plastics, containers, paints, coats, fibers and building materials. Alternatively, it can be used for protecting UV-sensitive contents, such as in filters, packaging materials, containers, paints, coats, inks, fibers, building materials, recording media, image displaying devices and solar cell covers, and can suppress the decomposition of compounds unstable to light.

The UV scattering agent of the invention may be dispersed in water, a hydrophilic organic solvent, a hydrophobic organic solvent or a mixed solvent thereof and used as a dispersion. The hydrophilic organic solvent and hydrophobic organic solvent are exemplified by the same solvents as mentioned above in connection with the polymer particle production method.

Here, in a dispersion obtained by adding 0.1 wt% of the UV scattering agent of the invention, the transmittance to UV having a wavelength of 360 nm is preferably less than 8%, more preferably less than 5%, and even more preferably less than 3%. When this UV scattering agent is compared with spherical polymer particles having the same composition and the same volume-equivalent diameter as polymer particle A, the UV-A and UV-B transmittances are preferably 1/2 or less. Here, UV-A, UV-B and UV-C refer to UV radiation having wavelengths of, respectively, 315 to 400 nm, 280 to 315 nm, and 200 to 280 nm.

The UV scattering agent of the invention may be used as an additive in shaped articles such as liquids, coats, films, sheet stock and paper. The UV scattering agent-containing composition of the invention may be widely used in, for example, light scattering agents and optical filter materials, colorants, cosmetics, absorbents, adsorbents, inks, electromagnetic shielding materials, fluorescence sensors, biological markers, recording media, recording devices, polarizing materials, drug supports for drug delivery systems (DDS), biosensors, DNA chips and diagnostic agents.

Using window glass products or interior decor products such as curtains and wall materials to block ultraviolet radiation from entering into a room, car or the like is useful not only for preventing sunburn and other adverse effects to the human body, but also for preventing the deterioration of decorative objects within the room or car.

The UV scattering agent of the invention is suitable as an additive for cosmetics because, in addition to having the low weight, light scattering properties, tactile qualities, flowability, solution dispersibility and the like inherent to elliptical and needle-like powder particle A, it allows the use of UV absorbers and other UV scattering agents to be eliminated or reduced. The fact that the UV scattering agent of the invention is not irritating to the skin also makes it useful as an additive for cosmetics. Moreover, the UV scattering agent of the invention, owing to its distinctive shape, has an adhesive strength differing from that of ordinary spherical products, and is effective for improving both the bonding strength of a pressed compacts of foundation or the like and the holding power following application. In addition, the optical characteristics make the skin appear lighter and can enhance the covering power due to a shading effect. Also, due to the slip properties particular to the particle shape, spreadability over the skin is excellent and furrows in the skin texture are finely filled, making wrinkles and pores inconspicuous, and the flowability of the overall product can be freely controlled. Also, advantage can be taken of the adhesive strength and holding power to increase the amount of polymer addition in the overall product, enabling the discovery of entirely new cosmetic effects. The amount of addition, based on the product contents, is preferably from 0.1 to 50 wt%, and more preferably 0.5 to 30 wt%. This amount may be suitably adjusted according to the intended use and purpose, such as improving the light scattering properties (e.g., the UV scattering effect and the shading effect), flowability, moldability and adhesion, and the finished look. According to studies by the inventors, as an additive for cosmetics, the addition of 1 to 20 wt% is especially preferred. Suitable adjustment and use in combination with commercial particles is also possible.

Cosmetics in which the inventive UV scattering agent has high UV-cutting and UV degradation-preventing effects are exemplified by skin care products, hair products, antiperspirants, makeup products, UV protection products and scented products. Examples include base cosmetics such as milky emulsions, creams, lotions, calamine lotion, sunscreens, makeup base, suntan lotions, aftershave lotions, preshave lotions, packs, cleansing materials, facial cleansers, cosmetics for acne, and essences; makeup cosmetics such as foundation, face powder, mascara, eye shadow, eyeliner, eyebrow, cheek, nail color, lip cream and lipstick; and also shampoos, rinses, conditioners, hair colors, hair tonics, setting agents, body powders, hair growth promoters, deodorants, depilatories, soaps, body shampoos, bath preparations, hand soaps and perfumes. The form of the product is not particularly limited, and includes, for example, liquids, emulsions, creams, solids, pastes, gels, powders, multi-layer preparations, mousses and sprays. Useful effects can be expected of the UV scattering agent as an additive in these cosmetics.

The UV scattering agent of the invention can be used as an additive for printing inks that may be used in, for example, screen printing, offset printing, process printing, gravure printing, pad printing, coaters and inkjet printing; an additive for writing implement inks in marking pens, ballpoint pens, fountain pens, calligraphy pens and magic markers; and an additive for writing materials such as crayons, artist's paints and erasers.

The UV scattering agent of the invention is suitable as an additive for paints that may be used in brush painting, spray painting, electrostatic spray painting, electrodeposition painting, flow coating, roller coating and dip coating. For example, it is suitable as an additive for paints and coatings that may be used on transportation equipment such as automobiles, railway cars, helicopters, ships, bicycles, snowmobiles, ropeways, lifts, hovercrafts and motorcycles; building members such as window sashes, shutters, cisterns, doors, balconies, outside panels for construction, roofing, staircases, skylights and concrete walls; the exterior walls and interior finish on the inside and outside of buildings; roadway members such as guardrails, pedestrian bridges, sound insulating walls, road signs, highway sidewalls, elevated railway bridges, and bridges; industrial plant members such as tanks, pipes, towers and smokestacks; agricultural facilities such as PVC and other types of greenhouses, silos and agricultural sheeting; telecommunications facilities such as utility poles, transmission towers and parabolic antennas; and electrical equipment such as electrical service boxes, lighting equipment, outdoor air conditioners, washing machines, refrigerators and electric ranges, as well as covers for these; and other articles such as monuments, gravestones, paving materials, windscreens, waterproof sheeting and curing sheets for construction.

The form of the paint is exemplified by not only solvent-based paints, but also water-dispersed paints, non-water-dispersed paints, powder paints and electrodeposition paints, and may be suitably selected as needed.

### EXAMPLES

Synthesis Examples, Working Examples of the invention and Comparative Examples are given below by way of illustration, although the invention is not limited to these Examples. In the evaluations within the Working Examples and Comparative Examples, measurement was carried out by the following methods. Reference examples are indicated with an asterisk *.

### (1) Aspect Ratio of Polymer Particles

A scanning electron microscope (S-4800, from Hitachi High Technologies Corporation) was used to capture photographs of the resulting elliptical or needle-like polymer particles at a magnification at which particle measurement is possible (300 to 30,000×) and, with the particles having been rendered into two-dimensional images (elliptical or needle-like polymer particles normally maintain a state in which the long axis direction is horizontally oriented), the length (L) and breadth (D) of each particle were measured, the aspect ratio (L/D) was calculated, and the average aspect ratio (P_{AV}) was determined.

The average length (L_{AV}) and average breadth (D_{AV}) of the particles were also calculated by repeatedly carrying out, at random, length (L) and breadth (D) measurements (n = 100).

### (2) Volume Mean Particle Size (MV) of Polymer Particles

This was measured using a MICROTRACK HRA9320-X100 (Nikkiso Co., Ltd.).

### [1] Synthesis of Elliptical or Needle-Like Polymer Particle A

### [Synthesis Example 1]

A polymethyl methacrylate particle solution was prepared by dissolving the compounds shown below in the respective phases, then mixing together and charging the water and oil phases into a 2,000 mL flask and heating and stirring (400 rpm) the mixture for about 8 hours at an oil bath temperature of 80°C and under a stream of nitrogen.

Water Phase:

| | |
|---|---|
| Water | 1,280.0 g |
| Polyvinylpyrrolidone (K-15) | 8.0 g |
| Ammonium persulfate | 4.8 g |

Oil Phase:

| | |
|---|---|
| Toluene | 80.0 g |
| Polystyrene | 16.0 g |
| Methyl methacrylate | 160.0 g |

(Polystyrene: from Sigma-Aldrich Co.; weight-average molecular weight, about 45,000)

Next, using a known suction filtration apparatus, this particle solution was repeatedly washed with methanol and filtered (5 times), then vacuum dried, giving Polymer Particle A1.

### [Synthesis Example 2]

Aside from changing the amount of polystyrene in the oil phase to 12.0 g, Polymer Particle A2 composed of polymethyl methacrylate was obtained in the same way as in Synthesis Example 1.

### [Synthesis Example 3]

Aside from changing the stirring speed to 500 rpm, Polymer Particle A3 composed of polymethyl methacrylate was obtained in the same way as in Synthesis Example 1.

### [Synthesis Example 4]

Aside from changing the stirring speed to 250 rpm, Polymer Particle A4 composed of polymethyl methacrylate was obtained in the same way as in Synthesis Example 1.

### [Synthesis Example 5]

Aside from changing the methyl methacrylate to styrene (Wako Pure Chemical Industries Co., Ltd.), Polymer Particle A5 composed of polystyrene was obtained in the same way as in Synthesis Example 1.

### [Synthesis Example 6]

A styrene-sodium p-styrenesulfonate copolymer particle solution was obtained by charging a 2,000 mL flask all at once with a mixture obtained by mixing together the compounds shown below in the indicated proportions, flushing out dissolved oxygen with nitrogen, and then heating and stirring for about 12 hours at an oil bath temperature of 88°C and under a stream of nitrogen.

| | |
|---|---|
| Styrene | 240 g |
| Sodium p-styrenesulfonate | 60 g |
| Butanol | 400 g |
| Methanol | 200 g |
| Water | 600 g |
| Azobisisobutyronitrile | 30 g |
| Polyvinylpyrrolidone (K-30) | 250 g |
| Sodium dodecylsulfonate | 6.0 g |

Next, using a known suction filtration apparatus, this particle solution was repeatedly washed with methanol and filtered (5 times), then vacuum dried, giving Polymer Particle A6.

### [Synthesis Example 7]

Aside from using styrene and 2-hydroxyethyl methacrylate as the copolymer components and setting the compositional ratio to 3:7 (weight ratio), Polymer Particle A7 composed of styrene-2-hydroxyethyl methacrylate copolymer was obtained in the same way as in Synthesis Example 6.

### [2] Synthesis of Particle B

### [Synthesis Example 8]

A particle dispersion was obtained by charging a 2,000 mL flask all at once with a mixture of the compounds shown below mixed in the indicated proportions, forming a suspension with a dispersion mixer at a dispersing blade speed of 1,000 rpm, then heating and stirring for 8 hours at an oil bath temperature of 80°C and under a stream of nitrogen. Next, centrifugal separation was carried out repeatedly (five times), followed by classification and washing operations, thereby giving spherical polymer particle B1 composed solely of polymethyl methacrylate and having an average particle size of 5 µm.

| | |
|---|---|
| Water | 533.3 g |
| Methyl methacrylate | 66.7 g |
| Lauryl peroxide | 3.33 g |
| Polyvinyl pyrrolidone (K-30) | 6.67 g |

### [Synthesis Example 9]

Aside from using styrene instead of methyl methacrylate, spherical polymer particle B2 composed solely of polystyrene and having an average particle size of 5 µm was produced in the same way as Synthesis Example 8.

### [Synthesis Example 10]

Aside from changing the amount of polyvinylpyrrolidone (K-30) used to 1/3, spherical polymer particle B3 composed solely of polymethyl methacrylate and having an average particle size of 150 µm was produced in the same way as in Synthesis Example 8.

### [Synthesis Example 11]

The particles obtained in Synthesis Example 1 were dissolved in toluene, formed into a sheet and re-dried, then passed through a grinder and subjected to a classifying operation, thereby producing pulverized (irregularly shaped) polymer particle B4 composed solely of polymethyl methacrylate and having an average particle size of 5 µm.

The MV, L_{AV}, D_{AV}, P_{AV}, ingredients and shapes of the particles obtained in Synthesis Examples 1 to 11 are summarized in Table 1.

**[Table 1]**

| | Polymer particle | MV (µm) | L_{AV} (µm) | D_{AV} (µm) | P_{AV} | Particle ingredients | Shape |
|---|---|---|---|---|---|---|---|
| Synthesis Example 1 | A1 | 7.1 | 20 | 3.6 | 6 | methyl methacrylate | elliptical/ needle-shaped |
| Synthesis Example 2 | A2 | 8.9 | 20 | 5.5 | 4 | methyl methacrylate | elliptical/ needle-shaped |
| Synthesis Example 3 | A3 | 3.5 | 10 | 1.5 | 8 | methyl methacrylate | elliptical/ needle-shaped |
| Synthesis Example 4 | A4 | 9.1 | 50 | 3.3 | 15 | methyl methacrylate | elliptical/ needle-shaped |
| Synthesis Example 5 | A5 | 8.3 | 21 | 3.8 | 7 | styrene | elliptical/ needle-shaped |
| Synthesis Example 6 | A6 | 8.1 | 20 | 4.0 | 6 | styrene sodium p-styrenesulfonate | elliptical/ needle-shaped |
| Synthesis Example 7 | A7 | 14.2 | 30 | 6.1 | 6 | styrene 2-hydroxyethyl methacrylate | elliptical/ needle-shaped |
| Synthesis Example 8 | B1 | 5.0 | - | - | 1.0 | methyl methacrylate | spherical |
| Synthesis Example 9 | B2 | 5.0 | - | - | 1.0 | styrene | spherical |
| Synthesis Example 10 | B3 | 150 | - | - | 1.0 | methyl methacrylate | spherical |
| Synthesis Example 11 | B4 | 5.0 | - | - | 1.3 | methyl methacrylate | pulverized (irregular) |

### [3] Production and Evaluation of Optical Measurement Dispersions

### [Examples 1 to 16, Comparative Examples 1 to 4]

Polymer particles A and B and purified water were mixed together in the proportions shown in Table 2 below to produce 0.1 wt% Polymer Particle Aqueous Dispersions 1 to 20.

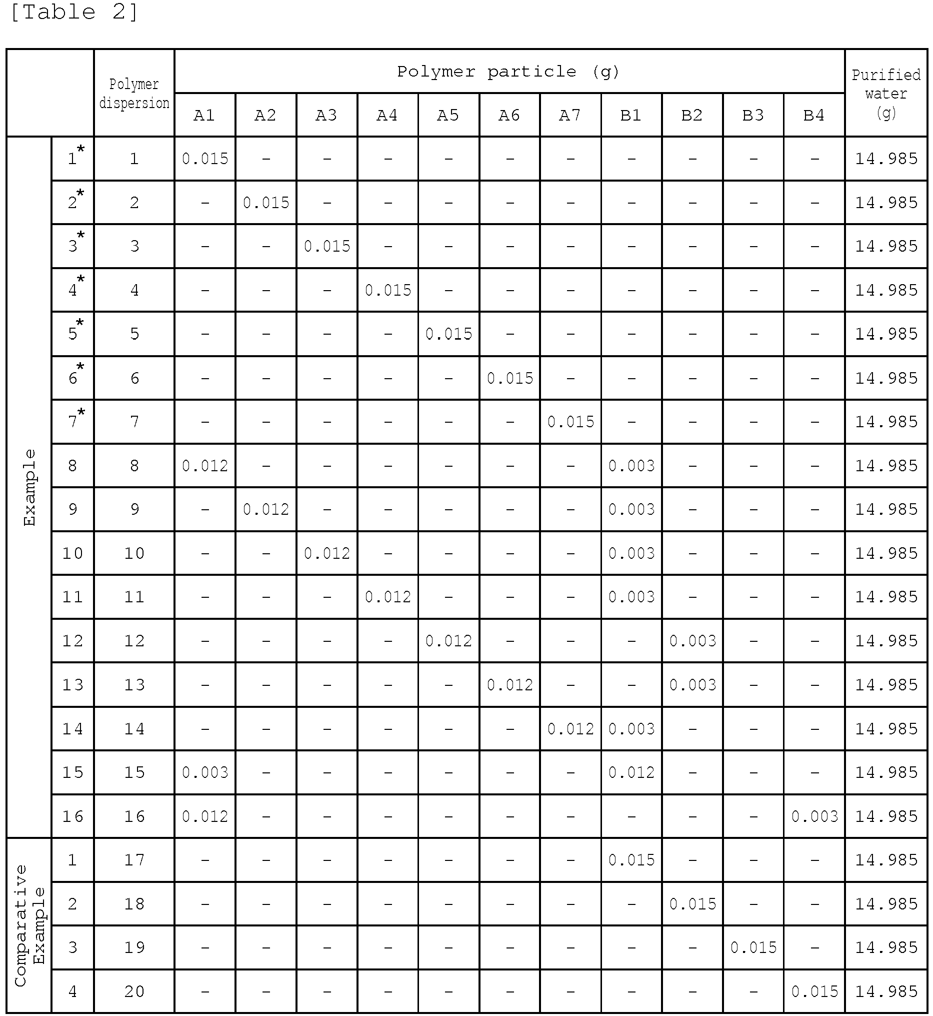

### [Evaluation Test 1]

Dispersions 1 to 20 were each poured into separate quartz cells (supplied with the following instrument) and, using a UV-visible spectrophotometer (UV-2450, from JASCO Corporation), UV transmission spectroscopy during particle dispersion was carried out at wavelengths of 320 nm, 360 nm and 400 nm. The results are shown in Table 3.

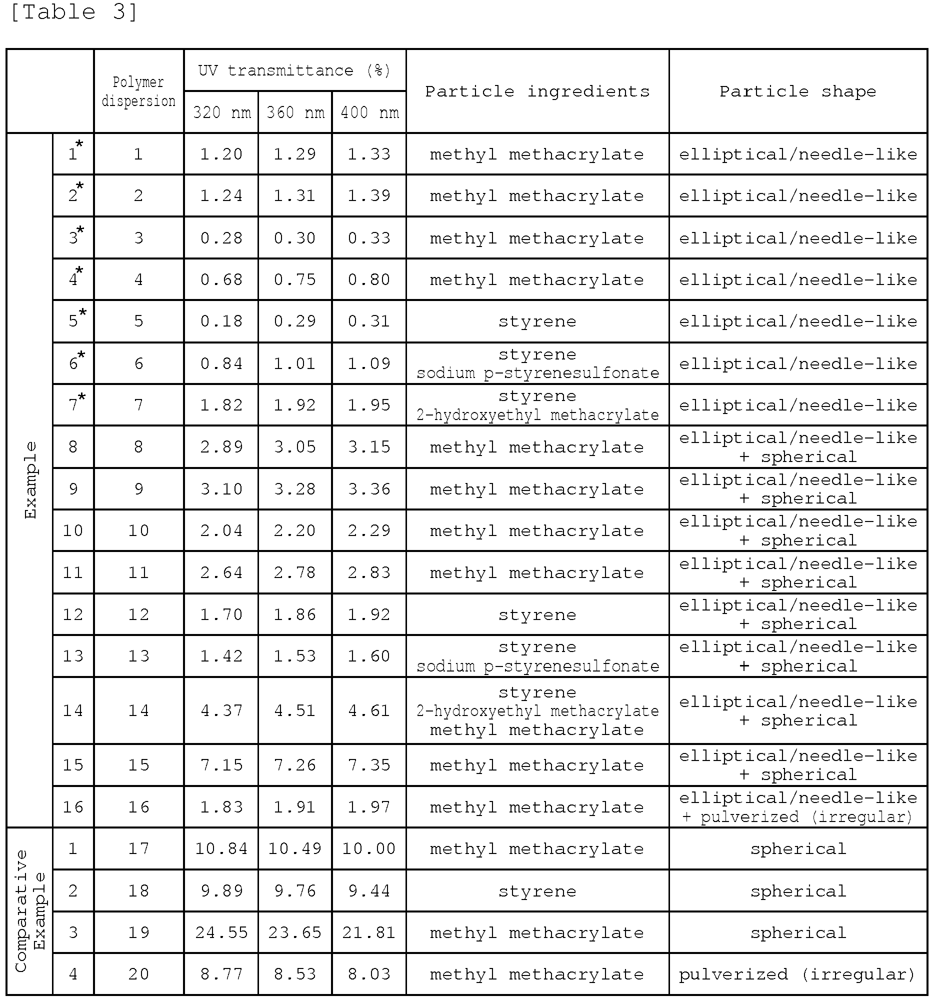

As a result of transmission spectroscopy, in a UV range that includes the UV-A region, elliptical or needle-like polymer particles were clearly demonstrated to have a high UV scattering effect.

### [4] Production and Evaluation of Optical Measurement Sheet

### [Examples 17 to 30, Comparative Examples 5 to 9]

A composition was prepared by mixing polymer particles A and B, a binder resin (PVA resin from Kuraray Co., Ltd.) and purified water in the proportions shown below in Table 4. The composition was coated onto one side of a 100 µm thick PET film (E-5000, from Toyobo Co., Ltd.) with a commercial bar coater. After coating, hot-air drying was carried out for 20 minutes in a dryer set to 50°C, following which an optical sheet was produced in such manner as to set the thickness of the coated layer to 40 µm.

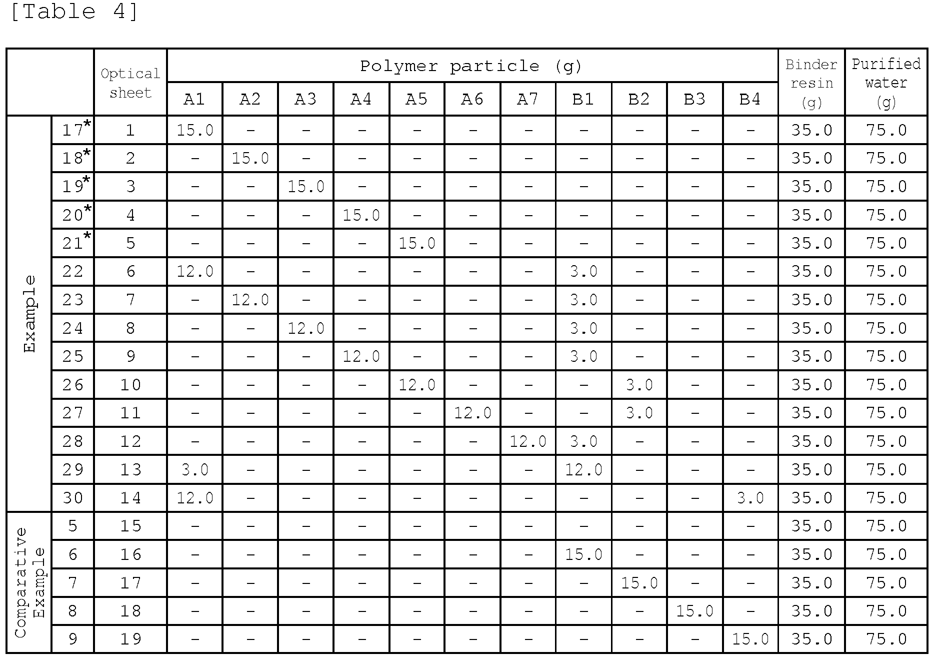

### [Evaluation Test 2]

UV transmission spectroscopy at wavelengths of 320 nm, 360 nm and 400 nm was carried out on Optical Sheets 1 to 19 with a UV-visible spectrophotometer (UV-2450, from JASCO Corporation). The results are shown in Table 5.

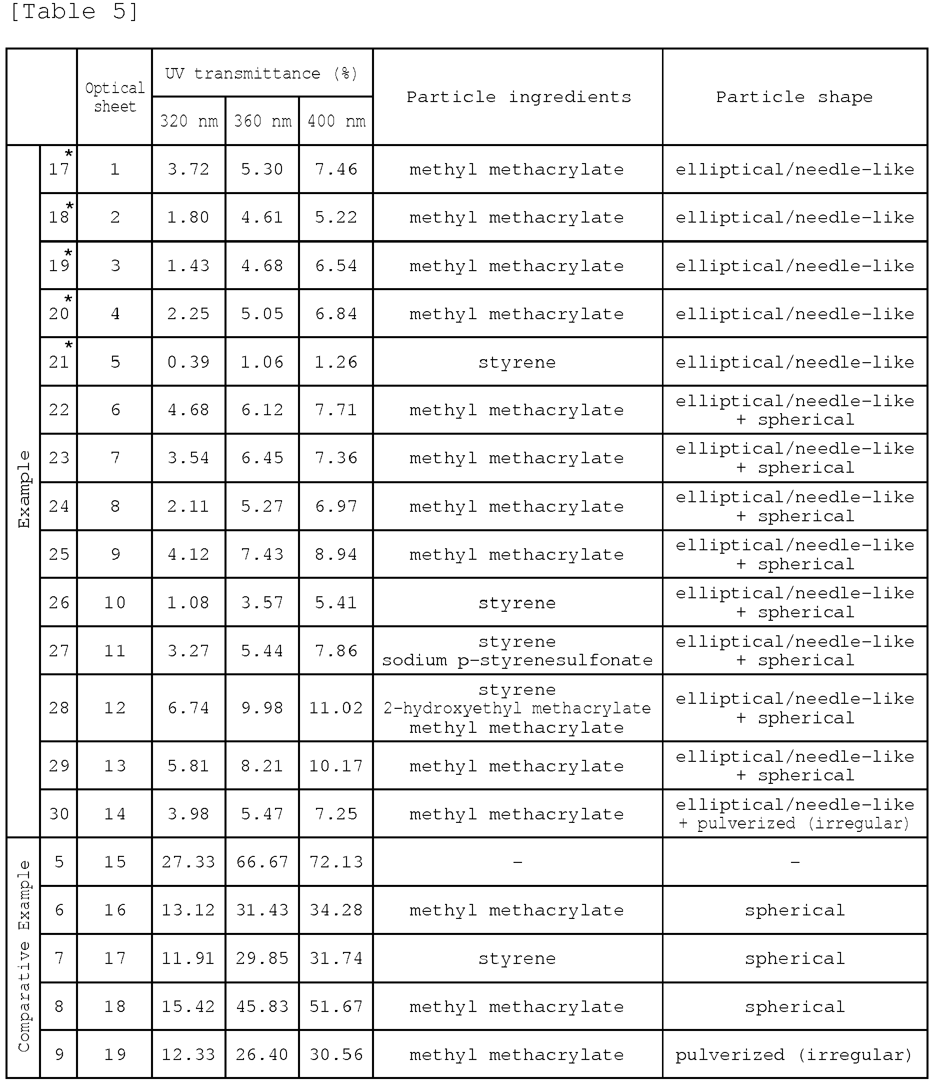

The results of transmission spectroscopy showed that transmitted light in the UV range (especially the UV-A region) is decreased, clearly demonstrating that additives containing the elliptical or needle-like polymer particles of the invention have a high UV scattering effect. The scattering effect in the visible light region was high as well, confirming that the hiding properties are also high.

When similar tests were carried out after producing a plurality of sheets (n = 5), the optical sheets in reference Examples 17 to 21 had high scattering effects but exhibited some degree of variability in the properties of each sheet compared with Examples 22 to 30. Such variability in the UV scattering effects is thought to be due to the flowability of the elliptical or needle-like polymer particles and the ready orientability of these particles. In other words, it was found that by mixing a second type of polymer particle having a different shape, such as a spherical or pulverized (irregular) shape, together with the elliptical or needle-like polymer particles (Examples 22 to 30), the second type of polymer particle gives rise to steric hindrance that suppresses orientation in the elliptical or needle-like polymer particles, thus enabling a UV scattering effect to be stably maintained without compromising the characteristic features of the elliptical or needle-like polymer particles.

In addition, on comparing Examples 22 and 29, it was demonstrated that when the elliptical or needle-like polymer particles are mixed together with spherical polymer particles composed of the same ingredients, reducing the proportion of elliptical or needle-like polymer particles lowers the UV scattering effects somewhat. However, compared with Comparative Example 6 (composed solely of spherical polymer of the same ingredients), the UV scattering effects of such a mixture were found to be sufficiently high.

### [Evaluation Test 3]

### [Examples 31 to 44, Comparative Examples 10 to 14]

UV scattering tests were carried out as follows using Optical Sheets 1 to 19.

Optical Sheets 1 to 19 cut to a size of 5 cm square were placed on and fixed to paper that changes color upon exposure to UV light (evaluation test paper, from Kenis, Ltd.) and then UV irradiated for 1 minute using a UV lamp, following which the degree of color change was determined from the appearance. The results are shown in Table 6.

The following UV lamps (Funakoshi Co., Ltd.) were used.
254 nm wavelength: MODEL UVG-54
302 nm wavelength: MODEL UVM-57
366 nm wavelength: MODEL UVL-56

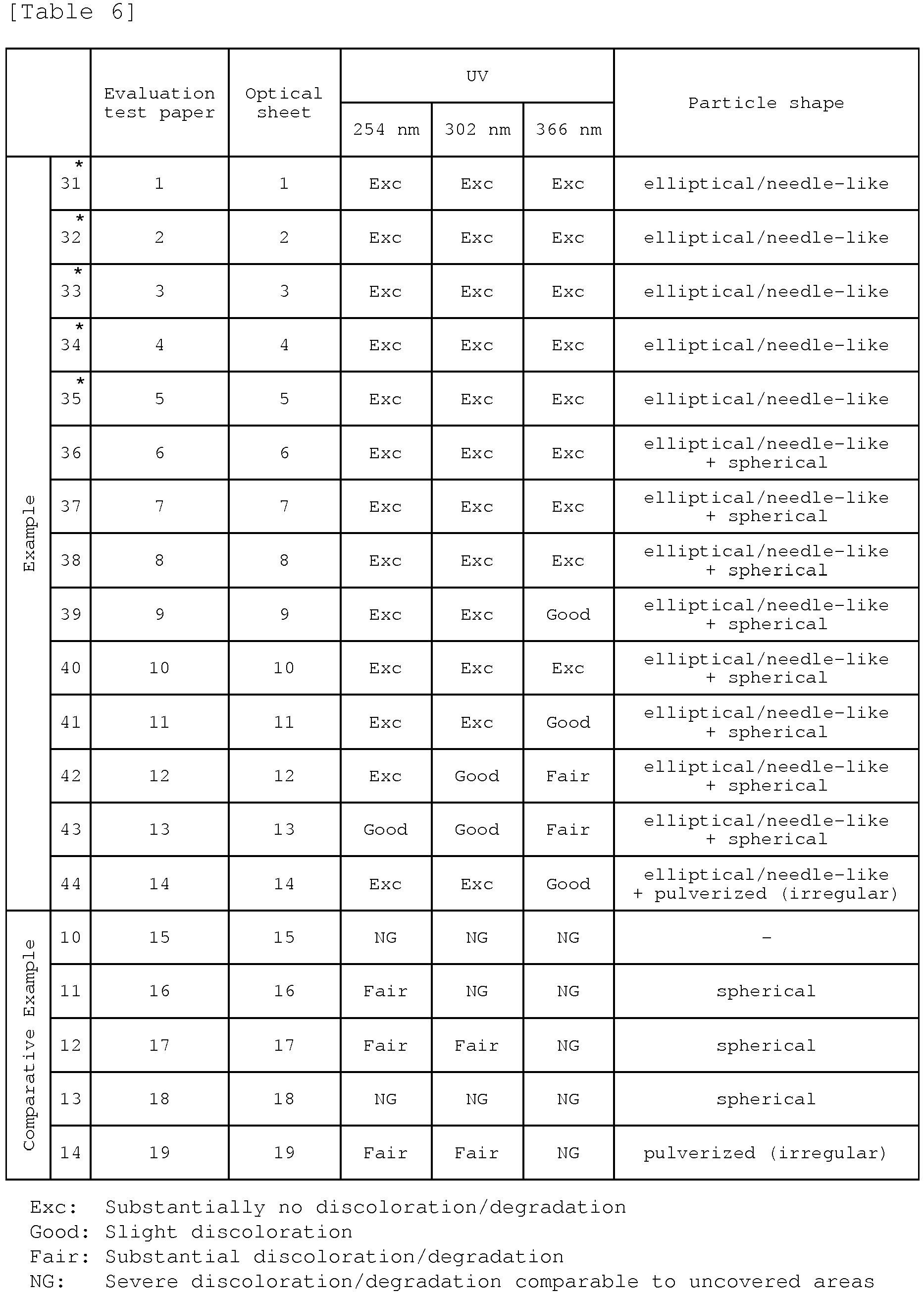

From the results of the UV scattering tests, it was confirmed that the elliptical or needle-like polymer particle-containing additive of the invention, when mixed into a base material or applied onto the surface layer of a base material, for example, has a UV scattering effect over the UV region (including UV-A, UV-B and UV-C) and is thereby able to suppress adverse effects such as UV-induced degradation and discoloration of cosmetics, inks, paints and colorants, etc. and damage to the health.

### [5] Evaluation of Reflected Light and Scattering Properties

### [Examples 45* and 46, Comparative Example 15]

Test sheets were prepared by uniformly applying (0.24 mg/cm²) the particles formulated in Example 19* onto black synthetic leather (5 cm × 8 cm) while patting with a cosmetic powder puff. Next, using an automated goniophotometer (GP-200, from Murakami Color Research Laboratory Co., Ltd.), a fixed amount of light was irradiated onto the test sheet at an incident angle of 45° and the light scattering distribution of the reflected light was measured (Example 45*). In addition, using the particles formulated in Example 24 and Comparative Example 6 instead of the particles formulated in Example 19, the light scattering distribution of reflected light was measured by the same method (Example 46, Comparative Example 15). The results are shown in FIG. 1.

FIG. 1 demonstrates that the light scattering effects of elliptical or needle-like polymer particles also are stably obtained without a loss in the UV scattering effects. Moreover, it was confirmed that these particles are effective in applications such as paints, inks, shaped articles, cosmetics and the like that require UV shielding.

### [6] Tests for Cosmetic Applications

### [Examples 47 to 60, Comparative Examples 16 to 19]

The particles formulated in Examples 17 to 30 and Comparative Examples 6 to 9 were evaluated as described below. The results are shown in Table 7.

Evaluated Qualities

| | |
|---|---|
| Feel: | The tactile feel of each type of particle when spread over the skin was evaluated. |
| Slip characteristics: | The slip characteristics were evaluated by placing 1 g of each type of particle on black synthetic leather, and measuring the length when spread with a finger. |
| Particle adhesion: | One gram of each type of particle was placed on black synthetic leather and uniformly spread with a powder puff, following which the leather was struck three times and the amount of particles remaining was examined with a digital microscope (VHX200, from Keyence Corporation). |

**[Table 7]**

| | | Particle | Feel | Slip | Particle adhesion |
|---|---|---|---|---|---|
| Example | 47* | Example 17 | Exc | Exc | Exc |
| | 48* | Example 18 | Exc | Exc | Exc |
| | 49* | Example 19 | Exc | Good | Exc |
| | 50* | Example 20 | Exc | Good | Exc |
| | 51* | Example 21 | Exc | Exc | Exc |
| | 52 | Example 22 | Exc | Exc | Exc |
| | 53 | Example 23 | Exc | Exc | Exc |
| | 54 | Example 24 | Exc | Exc | Exc |
| | 55 | Example 25 | Exc | Exc | Exc |
| | 56 | Example 26 | Exc | Exc | Exc |
| | 57 | Example 27 | Exc | Exc | Exc |
| | 58 | Example 28 | Good | Exc | Good |
| | 59 | Example 29 | Exc | Exc | Good |
| | 60 | Example 30 | Exc | Good | Exc |
| Comparative Example | 16 | Comparative Example 6 | Exc | Exc | NG |
| | 17 | Comparative Example 7 | Exc | Exc | NG |
| | 18 | Comparative Example 8 | NG | Good | NG |
| | 19 | Comparative Example 9 | NG | Good | Good |

The above results demonstrated that UV scattering effects are obtained without a loss in the feel, slip characteristics and particle adhesion of the elliptical or needle-like polymer particles.

Also, because it is effective for both UV scattering and visible light scattering, the UV scattering agent of the invention is effective also as a light scattering agent in hair care products, especially hair dyes.

### [Cosmetic Evaluation Test 1]

Foundations 1, 2 and 3 (oil-in-water emulsions) were prepared according to the compositions in Table 8 below.

**[Table 8]**

| Ingredients | Weight (g) | | |
|---|---|---|---|
| | Foundation 1* | Foundation 2 | Foundation 3 |
| 2% Acrylic acid-alkyl methacrylate copolymer dispersion | 15.0 | 15.0 | 15.0 |
| 2% Carboxyvinyl polymer dispersion | 15.0 | 15.0 | 15.0 |
| Dipropylene glycol | 5.0 | 5.0 | 5.0 |
| Disodium edetate | 0.05 | 0.05 | 0.05 |
| Purified water | 2.7 | 2.7 | 2.7 |
| Ethanol | 15.0 | 15.0 | 15.0 |
| Sorbitan monoisostearate | 2.0 | 2.0 | 2.0 |
| Polyoxyethylene(2) alkyl(C12-16) ether phosphate | 0.5 | 0.5 | 0.5 |
| 2-Ethylhexyl hydroxystearate | 5.0 | 5.0 | 5.0 |
| Methyl cyclopolysiloxane | 5.0 | 5.0 | 5.0 |
| 2-Amino-2-methyl-1-propanol | 0.5 | 0.5 | 0.5 |
| Titanium oxide | 10.0 | 10.0 | 10.0 |
| Red iron oxide | 0.2 | 0.2 | 0.2 |
| Yellow iron oxide | 1.0 | 1.0 | 1.0 |
| Black iron oxide | 0.1 | 0.1 | 0.1 |
| Talc | 3.7 | 3.7 | 3.7 |
| Crosslinked silicone powder | 1.5 | 1.5 | 1.5 |
| 2% Xantham gum dispersion | 15.0 | 15.0 | 15.0 |
| Phenoxyethanol | 0.2 | 0.2 | 0.2 |
| Sodium dehydroacetate | 0.05 | 0.05 | 0.05 |
| Polymer particles from Example 17* | 2.5 | - | - |
| Polymer particles from Example 22 | - | 2.5 | - |
| Polymer particles from Comparative Example 6 | - | - | 2.5 |

Ten people were selected as panelists, and the following five qualities were evaluated overall for Foundations 1, 2 and 3: "adhesion to skin," "sense of fit when applied," "feel during use," "soft focus effect" and "durability of cosmetic effect (4 hours)," based on which the acceptability of the cosmetic formulation was assessed.
A: <Foundation 1> was best
B: <Foundation 2> was best
C: <Foundation 3> was best
D: They were all the same

As a result, the assessments by the panelists were as follows:

### <Cosmetics for Use as Foundation>

A: 5 panelists
B: 4 panelists
C: 0 panelists
D: 1 panelist
Many of the panelists also thought that Foundations 1 and 2 had very similar properties.

### [Cosmetic Evaluation Test 2]

Foundations 4, 5, 6, 7 and 8 (powders) were produced according to the compositions shown in Table 9.

**[Table 9]**

| | Weight (g) | | | | |
|---|---|---|---|---|---|
| | Foundation 4* | Foundation 5* | Foundation 6 | Foundation 7 | Foundation 8 |
| Red iron oxide | 0.4 | 0.4 | 0.4 | 0.4 | 0.4 |
| Yellow iron oxide | 1.0 | 1.0 | 1.0 | 1.0 | 1.0 |
| Black iron oxide | 0.2 | 0.2 | 0.2 | 0.2 | 0.2 |
| Titanium oxide | 7.0 | 7.0 | 7.0 | 7.0 | 7.0 |
| Zinc oxide | 3.0 | 3.0 | 3.0 | 3.0 | 3.0 |
| Silicone-treated large particle-size titanium oxide | 3.0 | 3.0 | 3.0 | 3.0 | 3.0 |
| Lauroyl lysine powder | 14.0 | 14.0 | 14.0 | 14.0 | 14.0 |
| Titanium-mica powder | 4.0 | 4.0 | 4.0 | 4.0 | 4.0 |
| Talc | 35.97 | 35.97 | 35.97 | 35.97 | 35.97 |
| Methyl phenyl polysiloxane | 2.0 | 2.0 | 2.0 | 2.0 | 2.0 |
| Crystalline cellulose | 5.0 | 5.0 | 5.0 | 5.0 | 5.0 |
| Cornstarch | 10.0 | 10.0 | 10.0 | 10.0 | 10.0 |
| Methyl paraben | 0.1 | 0.1 | 0.1 | 0.1 | 0.1 |
| Sodium dehydroacetate | 0.1 | 0.1 | 0.1 | 0.1 | 0.1 |
| Liquid paraffin | 1.5 | 1.5 | 1.5 | 1.5 | 1.5 |
| Butylene glycol | 0.5 | 0.5 | 0.5 | 0.5 | 0.5 |
| Job's tears extract | 0.1 | 0.1 | 0.1 | 0.1 | 0.1 |
| Ginseng root extract | 0.1 | 0.1 | 0.1 | 0.1 | 0.1 |
| Ubiquinone | 0.03 | 0.03 | 0.03 | 0.03 | 0.03 |
| * Polymer particles from Example 19* | 12.0 | - | - | - | - |
| Polymer particles from Example 20* | - | 12.0 | - | - | - |
| Polymer particles from Example 24 | - | - | 12.0 | - | - |
| Polymer particles from Example 25 | - | - | - | 12.0 | - |
| Polymer particles from Comparative Example 6 | - | - | - | - | 12.0 |

Ten people were selected as panelists, and the following five qualities were evaluated overall for Foundations 4, 5, 6, 7 and 8: "adhesion to skin," "sense of fit when applied," "feel during use," "soft focus effect" and "durability of cosmetic effect (4 hours)," based on which the acceptability of the cosmetic formulation was assessed.
A: <Foundation 4> was best
B: <Foundation 5> was best
C: <Foundation 6> was best
C: <Foundation 7> was best
E: <Foundation 8> was best
F: They were all the same

As a result, the assessments by the panelists were as follows:

### <Cosmetics for Use as Foundation>

A: 2 panelists
B: 3 panelists
C: 2 panelists
D: 3 panelists
E: 0 panelists
F: 0 panelists
Moreover, many of the panelists thought that Foundations 4, 5, 6 and 7 had very similar properties. Many of the panelists also thought that Foundations 4, 5, 6 and 7 were particularly outstanding with respect to "adhesion to skin," "soft focus effect" and "durability of cosmetic effect (4 hours)." On the other hand, many felt that Foundation 8 was lacking in "adhesion to skin" and "durability of cosmetic effect (4 hours)."

### [Cosmetic Evaluation Test 3]

Mascara Cosmetics 1, 2 and 3 (cosmetics for use as mascara) were produced according to the compositions in Table 10 below.

**[Table 10]**

| Ingredients | Weight (g) | | |
|---|---|---|---|
| | Cosmetic Mascara 1* | Cosmetic Mascara 2 | Cosmetic Mascara 3 |
| Hydrogenated polyisobutene | 47 | 47 | 47 |
| Cyclomethicone | 10.5 | 10.5 | 10.5 |
| Dextrin palmitate | 7.0 | 7.0 | 7.0 |
| Sucrose acetate/stearate | 5.0 | 5.0 | 5.0 |
| Disteardionium hectorite | 5.0 | 5.0 | 5.0 |
| Tris(trimethylsiloxy)silyl propyl carbamate pullulan | 4.5 | 4.5 | 4.5 |
| Candelilla wax | 4.0 | 4.0 | 4.0 |
| PEG-10 dimethicone | 3.0 | 3.0 | 3.0 |
| Butylene glycol | 1.5 | 1.5 | 1.5 |
| Purified water | 1.5 | 1.5 | 1.5 |
| Triisostearic acid PEG-20 hydrogenated castor oil | 1.0 | 1.0 | 1.0 |
| Hydrophobic-treated pigment | 5.0 | 5.0 | 5.0 |
| Nylon fibers (1-3 mm) | 3.0 | 3.0 | 3.0 |
| * Polymer particles from Example 17 | 2.0 | - | - |
| Polymer particles from Example 22 | - | 2.0 | - |
| Polymer particles from Comparative Example 6 | - | - | 2.0 |

Ten people were selected as panelists, and the following four qualities were evaluated overall for Mascara Cosmetics 1, 2 and 3: "mascara adherence," "mascara sense of volume," "mascara appearance" and "durability of cosmetic effect (4 hours," based on which the acceptability of the cosmetic formulation was assessed.
A: <Mascara Cosmetic 1> was best
B: <Mascara Cosmetic 2> was best
C: <Mascara Cosmetic 3> was best
D: They were all the same

As a result, the assessments by the panelists were as follows:

### <Cosmetic for Use as Mascara>

A: 5 panelists
B: 4 panelists
C: 0 panelists
D: 1 panelist
Many of the panelists also thought that Mascara Cosmetics 1 and 2 had very similar properties.

The above results demonstrate that the UV scattering agent of the invention, while maintaining UV scattering properties, is useful also as an additive (ingredient) for makeup, skin care products and cosmetics in general.

Because they are useful both for scattering UV radiation and also scattering visible light, the UV scattering agents of the invention are useful also as light scattering agents for hair care products, especially hair dyes.

## Claims

1. An ultraviolet scattering agent **characterized by** comprising at least one type of elliptical or needle-like polymer particle A, wherein
(1) a projected two-dimensional image obtained by irradiating the particle with light from a direction perpendicular to a long axis of the particle has a length (L) the average (L_{AV}) of which is 0.1 to 80 µm,
(2) a projected two-dimensional image obtained by irradiating the particle with light from a direction perpendicular to a long axis of the particle has a breadth (D) the average (D_{AV}) of which is 0.05 to 40 µm, and
(3) the average (P_{AV}) of the aspect ratio (L/D) calculated from the length (L) and breadth (D) is from 2 to 30; and
**characterized in that** the ultraviolet scattering agent further comprises at least one type of particle B which has
(4) a shape differing from that of polymer particle A and a volume mean particle size (MV_{B}) that satisfies the condition 1/5 × D_{AV} ≤ MV_{B} ≤ L_{AV}.

2. The ultraviolet scattering agent of claim 1, wherein polymer particle A is made of at least one type of resin selected from the group consisting of styrene resins, (meth)acrylic resins, vinyl ester resins, poly-N-vinyl compound resins, polyolefin resins, polydiene resins, polyester resins, silicone resins, polyurethane resins, polyamide resins, polyimide resins, epoxy resins, polyvinyl butyral resins, phenolic resins, amino resins, oxazoline resins and carbodiimide resins.

3. The ultraviolet scattering agent of claim 1 or 2, wherein polymer particle A is made of a (co)polymer comprising at least one type of monomer selected from the group consisting of styrenes, (meth)acrylic acids, (meth)acrylic esters, vinyl esters, N-vinyl compounds, olefins, fluorinated olefins and conjugated dienes.

4. The ultraviolet scattering agent of claim 1, wherein particle B is a polymer particle.

5. The ultraviolet scattering agent of any one of claims 1 to 4, wherein particle B is spherical or substantially spherical.

6. The ultraviolet scattering agent of any one of claims 1 to 5, wherein the weight ratio of polymer particle A to particle B is between 99:1 and 10:90.

7. A resin composition comprising the ultraviolet scattering agent of any one of claims 1 to 6.

8. A dispersion comprising the ultraviolet scattering agent of any one of claims 1 to 6.

9. A paint comprising the ultraviolet scattering agent of any one of claims 1 to 6.

10. An ink comprising the ultraviolet scattering agent of any one of claims 1 to 6.

11. A cosmetic composition comprising the ultraviolet scattering agent of any one of claims 1 to 6.

12. A shaped article comprising the ultraviolet scattering agent of any one of claims 1 to 6.

13. The shaped article of claim 12 which is a film, sheet or paper.

14. A method for imparting ultraviolet shielding properties by adding the ultraviolet scattering agent of any one of claims 1 to 6 to a transparent or translucent resin, water or a volatile oil.

## Patentansprüche

1. Ultraviolettstreuungsmittel, das **dadurch gekennzeichnet ist, dass** es zumindest eine Art von elliptischem oder nadelartigem Polymerteilchen A umfasst, wobei
(1) ein projiziertes zweidimensionales Bild erhalten wird, indem das Teilchen mit Licht bestrahlt wird, das aus einer auf eine lange Achse eines Teilchens senkrechten Richtung kommt, das eine Länge (L) aufweist, dessen Mittel (L_{AV}) 1,0 bis 80 µm beträgt,
(2) ein projiziertes zweidimensionales Bild erhalten wird, indem das Teilchen mit Licht bestrahlt wird, das aus einer auf eine lange Achse eines Teilchens senkrechten Richtung kommt, das eine Breite (D) aufweist, dessen Mittel (D_{AV}) 0,05 bis 40 µm beträgt, und
(3) das Mittel (P_{AV}) des Seitenverhältnisses (L/D), das aus der Länge (L) und der Breite (D) berechnet wird, 2 bis 30 beträgt; und
**dadurch gekennzeichnet, dass** das Ultraviolettstreuungsmittel weiters zumindest eine Art von Teilchen B umfasst, das
(4) eine Form hat, die von Polymerteilchen A abweicht, und eine mittlere Teilchenvolumengröße (MV_{B}) aufweist, die die Bedingung 1/5 x D_{AV} ≤ MV_{B} ≤ L_{AV} erfüllt.

2. Ultraviolettstreuungsmittel nach Anspruch 1, wobei Polymerteilchen A aus zumindest einer Art von Harz hergestellt ist, das aus der aus Styrolharzen, (Meth)acrylharzen, Vinylesterharzen, Poly-N-Vinyl-Verbundharzen, Polyolefinharzen, Polydienharzen, Polyesterharzen, Siliconharzen, Polyurethanharzen, Polyamidharzen, Polyimidharzen, Epoxidharzen, Polyvinylbutyralharzen, Phenolharzen, Aminoharzen, Oxazolinharzen und Carbodiimidharzen bestehenden Gruppe ausgewählt ist.

3. Ultraviolettstreuungsmittel nach Anspruch 1 oder 2, wobei Polymerteilchen A aus einem (Co-)Polymer hergestellt ist, das zumindest eine Art von Monomer umfasst, das aus der aus Styrolen, (Meth)acrylsäuren, (Meth)acrylsäureestern, Vinylestern, N-Vinylverbindungen, Olefinen, fluorierten Olefinen und konjugierten Dienen bestehenden Gruppe ausgewählt ist.

4. Ultraviolettstreuungsmittel nach Anspruch 1, wobei Teilchen B ein Polymerteilchen ist.

5. Ultraviolettstreuungsmittel nach einem der Ansprüche 1 bis 4, wobei Teilchen B kugelförmig oder im Wesentlichen kugelförmig ist.

6. Ultraviolettstreuungsmittel nach einem der Ansprüche 1 bis 5, wobei das Gewichtsverhältnis von Polymerteilchen A zu Teilchen B zwischen 99:1 und 10:90 liegt.

7. Harzzusammensetzung, die das Ultraviolettstreuungsmittel nach einem der Ansprüche 1 bis 6 umfasst.

8. Dispersion, die das Ultraviolettstreuungsmittel nach einem der Ansprüche 1 bis 6 umfasst.

9. Farbe, die das Ultraviolettstreuungsmittel nach einem der Ansprüche 1 bis 6 umfasst.

10. Druckfarbe, die das Ultraviolettstreuungsmittel nach einem der Ansprüche 1 bis 6 umfasst.

11. Kosmetische Zusammensetzung, die das Ultraviolettstreuungsmittel nach einem der Ansprüche 1 bis 6 umfasst.

12. Geformter Artikel, der das Ultraviolettstreuungsmittel nach einem der Ansprüche 1 bis 6 umfasst.

13. Geformter Artikel nach Anspruch 12, der ein Film, Bogen oder Papier ist.

14. Verfahren zur Verleihung von Ultraviolett-Abschirmeigenschaften durch die Zugabe des Ultraviolettstreuungsmittels nach einem der Ansprüche 1 bis 6 zu einem transparenten oder durchsichtigen Harz, Wasser oder ätherischen Öl.

## Revendications

1. Agent diffusant les ultraviolets **caractérisé en ce qu'**il comprend au moins une particule polymère elliptique ou aciculaire A, dans lequel
(1) une image bidimensionnelle projetée, obtenue par irradiation de la particule avec une lumière provenant d'une direction perpendiculaire à l'axe long de la particule, a une longueur (L) dont la moyenne (L_{AV}) est de 0,1 à 80 µm,
(2) une image bidimensionnelle projetée, obtenue par irradiation de la particule avec une lumière provenant d'une direction perpendiculaire à l'axe long de la particule, a une largeur (D) dont la moyenne (D_{AV}) est de 0,05 à 40 µm, et
(3) la moyenne (P_{AV}) du rapport d'aspect (L/D), calculé à partir de la longueur (L) et de la largeur (D), est de 2 à 30 ; et
**caractérisé en ce que** l'agent diffusant les ultraviolets comprend en outre au moins un type de particule B qui a
(4) une forme différant de celle de la particule polymère (A) et une granulométrie moyenne en volume (MV_{B}) qui satisfait à la condition 1/5 x D_{AV} ≤ MV_{B} ≤ L_{AV}.

2. Agent diffusant les ultraviolets selon la revendication 1, dans lequel la particule polymère A est faite d'au moins un type de résine choisie dans le groupe constitué par les résines styréniques, les résines (méth)acryliques, les résines d'ester vinylique, les résines de composés poly-N-vinylique, les résines de polyoléfine, les résines de polydiène, les résines de polyester, les résines de silicone, les résines de polyuréthane, les résines de polyamide, les résines de polyimide, les résines époxy, les résines de poly(butyral de vinyle), les résines phénoliques, les résines amino, les résines d'oxazoline et les résines de carbodiimide.

3. Agent diffusant les ultraviolets selon la revendication 1 ou 2, dans lequel la particule polymère A est faite d'un (co)polymère comprenant au moins un type de monomère choisi dans le groupe constitué par les styrènes, les acides (méth)acryliques, les esters (méth)acrylates, les esters vinyliques, les composés N-vinyliques, les oléfines, les oléfines fluorées et les diènes conjugués.

4. Agent diffusant les ultraviolets selon la revendication 1, dans lequel la particule B est une particule polymère.

5. Agent diffusant les ultraviolets selon l'une quelconque des revendications 1 à 4, dans lequel la particule B est sphérique ou pratiquement sphérique.

6. Agent diffusant les ultraviolets selon l'une quelconque des revendications 1 à 5, dans lequel le rapport en poids de la particule polymère A à la particule B est compris entre 99/1 et 10/90.

7. Composition de résine comprenant l'agent diffusant les ultraviolets de l'une quelconque des revendications 1 à 6.

8. Dispersion comprenant l'agent diffusant les ultraviolets de l'une quelconque des revendications 1 à 6.

9. Peinture comprenant l'agent diffusant les ultraviolets de l'une quelconque des revendications 1 à 6.

10. Encre comprenant l'agent diffusant les ultraviolets de l'une quelconque des revendications 1 à 6.

11. Composition cosmétique comprenant l'agent diffusant les ultraviolets de l'une quelconque des revendications 1 à 6.

12. Article façonné comprenant l'agent diffusant les ultraviolets de l'une quelconque des revendications 1 à 6.

13. Article façonné selon la revendication 12, qui est un film, une feuille ou un papier.

14. Procédé pour conférer des propriétés de blindage aux ultraviolets par addition de l'agent diffusant les ultraviolets de l'une quelconque des revendications 1 à 6 à une résine transparente ou translucide, à de l'eau ou à une huile volatile.
